# EUROPEAN PATENT APPLICATION

(11) **EP 2 036 578 A1**
(43) Date of publication of application: **18.03.2009**
(21) Application number: 07075805.7
(22) Date of filing: 13.09.2007
(51) Int. Cl.: A61K 51/04

(54) **Diagnostic agents for positron emission imaging using F-18 radio labeled amino-alcohols**

(71) Applicant: Bayer Schering Pharma Aktiengesellschaft, 13353 Berlin (DE)
(72) Inventor: Berndt, Mathias, 12163 Berlin (DE); Berndorff, Dietmar, 13467 Berlin (DE); Lehmann, Lutz, 13357 Berlin (DE); Zitzmann-Kolbe, Sabine, 10551 Berlin (DE)

(57) **Abstract**

This invention relates to novel compounds F-18 radio-labeled amino-alcohols suitable for labeling or already labeled by ¹⁸F methods of preparing such a compound, compositions comprising such compounds, kits comprising such compounds or compositions and uses of such compounds, compositions or kits for diagnostic imaging by positron emission tomography (PET).

## Description

### Field of Invention

This invention relates to novel compounds suitable for labeling or already labeled by ¹⁸F methods of preparing such a compound, compositions comprising such compounds, kits comprising such compounds or compositions and uses of such compounds, compositions or kits for diagnostic imaging by positron emission tomography (PET).

### Background

Molecular imaging has the potential to detect disease progression or therapeutic effectiveness earlier than most conventional methods in the fields of oncology, neurology and cardiology. Of the several promising molecular imaging technologies having been developed as optical imaging and MRI, PET is of particular interest for drug development because of its high sensitivity and ability to provide quantitative and kinetic data.

Positron emitting isotopes include carbon, nitrogen, and oxygen. These isotopes can replace their non-radioactive counterparts in target compounds to produce tracers that function biologically and are chemically identical to the original molecules for PET imaging. On the other hand, ¹⁸F is the most convenient labeling isotope due to its relatively long half life (109.6 min) which permits the preparation of diagnostic tracers and subsequent study of biochemical processes. In addition, its low β+ energy (635 keV) is also advantageous.

The aliphatic ¹⁸F-fluorination reaction is of great importance for ¹⁸F-labeled radiopharmaceuticals which are used as *in vivo* imaging agents targeting and visualizing diseases, e.g. solid tumours or diseases of brain. A very important technical goal in using ¹⁸F-labelled radiopharmaceuticals is the quick preparation and administration of the radioactive compound due to the fact that the ¹⁸F isotopes have a short half-life of about only 111 minutes.

The best known example for PET imaging of diseases is 2-[¹⁸F]fluorodeoxyglucose ([¹⁸F]FDG), which is the most widely used PET radiopharmaceutical [J Nucl Med (1978), 19: 1154-1161].

However, a number of pitfalls and artefacts have been ascribed to FDG imaging and more continue to surface as the worldwide experience with FDG increases. The area most common for interpretative pitfalls with FDG is related to uptake in active skeletal muscle (Seminars in Nuclear Medicine, (2004), XXXIV, 2, pp.122-133). Specifically, laryngeal muscle activity should be minimized in patients with head and neck cancers, requiring silence before and after injection. It is particularly important that the patient be relaxed prior to scanning to reduce or eliminate the showing of activated brown fat or "muscle tension." Even when recognized as a benign variant, the degree of uptake may obscure malignant lymphadenopathy in that region. It is common to starve the patient for 4-6 hours before injection keeping insulin levels low to minimize uptake into muscle, fat and myocardium. Some other negatives associated with FDG imaging include moderate uptake of glandular breast tissue, resulting in decreased ability to diagnose low-grade breast cancer. Uptake within the gastrointestinal tract and the myocardium can also be highly variable. As mentioned previously, many benign conditions can cause high accumulation of FDG creating the potential for false positive interpretation. Most of these artefacts are related to inflammatory, infective or granulomatous processes (Seminars in Nuclear Medicine, (2004), XXXIV, 2, pp.122-133" Seminars in Nuclear Medicine, (2004), XXXIV, 1, pp.56-69, (2004), J Nucl Med (2004), 45, pp. 695-700). Other tumors including mucosal associated lymphomas, small lymphocytic cell lymphoma, some neuroendocrine tumors, sclerotic bone metastases and renal cell carcinomas can be virtually inconspicuous due to low uptake or higher neighbouring background activity. Specifically related to PET-CT are pitfalls associated with breathing pattern differences between modalities, despite dedicated combined scanners (Seminars in Nuclear Medicine, (2004), XXXIV, 2, pp.122-133). CT scans are acquired during breath hold, while FDG scans are taken during tidal breathing resulting in potential mis-registration of pulmonary nodules between the modalities as much as 15 mm at the periphery and base of the lungs.

Especially for the PET imaging of prostate cancer, but also for other type of cancers, tetra-substituted ammonium derivatives labeled with C-11 and F-18 isotopes and based on choline structure have been published and claimed (e.g. JP09048747A, WO2001/082864A2, US 2002061279A1).

Among these derivatives [methyl-(C-11)]choline (CH), [F-18]fluorocholine (FCH), [F-18]fluoroethylcholine (FEC), [F-18]fluoromethylethylcholine (FMEC) and [F-18]fluoropropylcholine (FPC) are the best investigated compounds (see Fig 1; e.g. DeGrado et al. Nuclear Medicine (2001), 42(12), 1805-1814).

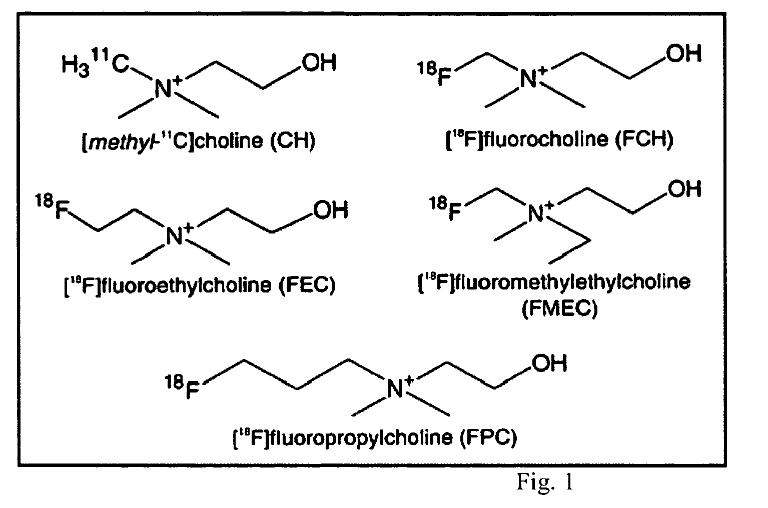

The enrichment of these above-mentioned compounds in tumors is acceptable for clinical use and for the diagnostic analysis of diseases. Nevertheless it would be useful to have [F-18]-tracers available which show higher enrichment in tumors and/or an imaging of cancer tissue in a more specific manner.

Recently, Ponde and Mintz reported on [C-14]-tracers of ethanol amine. Cancer cell uptake *in-vitro* data demonstrated that the [C-14] derivative of ethanolamine (**1**) and the [C-14] derivative of *N,N*-dimethyl-ethanolamine (2) (see Fig. 2) showed 2-3 fold higher uptake than [C-14]choline (Ponde et al. J. Nucl. Med. 2007, meeting abstract, 48, p. 33). Bio-distribution studies demonstrated tumor uptake of [C-14]ethanolamine and [C-14]dimethyl-ethanolamine.

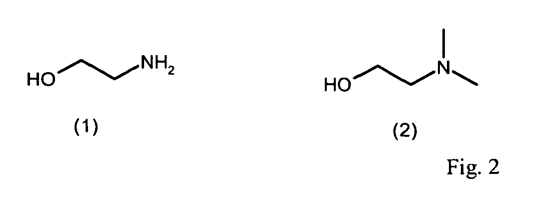

For the synthesis of [F-18] labeled compounds it is necessary to react the [F-18]-fluoride anion with a molecule comprising a leaving group. Amino-alcohols comprising leaving groups are well-known, e.g. methanesulfonic acid 2-[benzooxazol-2-yl-(2-hydroxy-ethyl)-amino]-ethyl ester (J. Labelled Compd. Radiopharm.; 44; 2001; S316 - S318) and toluene-4-sulfonic acid 2-{phenyl-[2-(tetrahydro-pyran-2-yloxy)-ethyl]-amino-ethyl ester (Org. Lett. 2005, 2857-2859) has been recently described. Methanesulfonic acid 2-{[2-(tert-butyldimethyl-silanyloxy)-ethyl]-(5-carbamoyl-2,4-dinitro-phenyl)-amino}-ethyl ester (J. Med. Chem. 37, 14, 1994, 2175-2184) is another example for such a molecule. Amino-alcohols comprising leaving groups can be protected e.g. as 4,5-dihydro-oxazoles (see compound 3 and 5, J. Carbohydr. Chem.; 24; 2; 2005; 103 - 130; WO2003/99195A2, J. Org. Chem.; 54; 6; 1989; 1295-1304) or as N-Boc-O-benzyl derivatives (see compound 4, EP 1679296 A1).

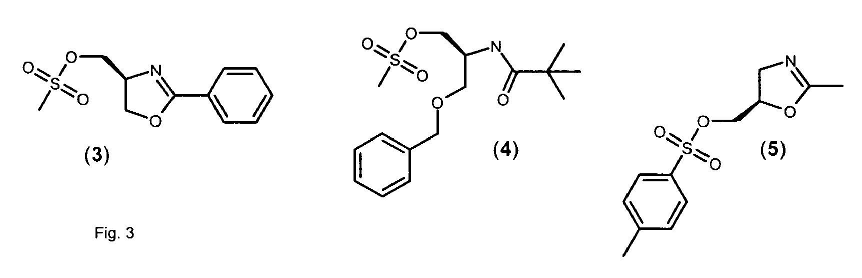

Aim of the invention is to find new compounds which emit positrons and which allow the imaging of diseases by [F-18]-PET imaging methods preferably of tumors and its mestatasis.

### Summary of the Invention

(an overview on aspects of the present invention is shown in Fig. 4):
■ The present invention provides novel compounds of Formulae I, II, III, IX, and X or pharmaceutically acceptable salts of an inorganic or organic acid thereof, hydrates, complexes, esters, amides, solvates and prodrugs thereof.
■ The present invention also provides pharmaceutical compositions comprising a radiolabeled compound of Formula II or pharmaceutically acceptable salts of an inorganic or organic acid thereof, hydrates, complexes, esters, amides, solvates and prodrugs thereof and a optionally a pharmaceutically acceptable carrier, diluent, adjuvant or excipient.
■ The present invention provides a pharmaceutical composition comprising a compound of Formula X or a compound of Formula XI, which are precursor (starting material) for PET-isotope labeling compounds of Formula II.
■ The present invention provides a method of imaging diseases, the method comprising introducing into a patient a detectable quantity of a labeled compound of Formula II or pharmaceutically acceptable salts of an inorganic or organic acid thereof, hydrates, complexes, esters, amides, solvates and prodrugs thereof.
■ The present invention is directed to the use of compounds of Formula I, II, III or IX for the manufacture of a pharmaceutical for imaging cancer and more preferably prostate cancer and its metastases.
■ The present invention provides the compounds of Formula II for use as medicament.
■ The present invention is directed to the use of compounds of Formula I, II, III or IX for the manufacture of a pharmaceutical for treating cancer and more preferably prostate cancer and its metastases.
■ The present invention also provides methods for producing compounds of Formula III by reacting
   a) compounds of Formula I with [¹⁸F]-fluoride,
   b) compounds of Formula IV, being generated from compounds of Formula V, with compounds of Formula VI or
   c) compounds of Formula VII with [¹⁸F]-fluoride.
■ The invention also provides methods for producing compounds of Formula IX by reacting
   a) compounds of Formula X with [¹⁸F]-fluoride
   b) compounds of Formula XI with [¹⁸F]-fluoride
■ The invention also provides methods for producing compounds of Formula II by reacting
   a) compounds of Formula III with deprotecting agent(s);
   b) compounds of Formula IV, being generated from compounds of Formula V, with a compound of Formula VIII;
   c) compounds of Formula IX being generated from compounds of Formula X or compounds of Formula XI with deprotecting agents;
■ The present invention also provides a kit for preparing a radiopharmaceutical preparation, said kit comprising a sealed vial containing a predetermined quantity of
   o the compound of Formula I;
   o the compounds of Formula V and VI;
   o the compounds of Formula V and VIII;
   o the compounds of Formula VII;
   o the compounds of Formula X or
   o the compounds of Formula XI.
■ A further aspect of this invention is directed to methods and intermediates useful for producing the imaging compounds of Formula I, II, III and IX described herein. More specifically the compounds of this invention are useful for the imaging of a variety of cancers including but not limited to: carcinoma such as bladder, breast, colon, kidney, liver, lung, including small cell lung cancer, esophagus, gall-bladder, ovary, pancreas, stomach, cervix, thyroid, prostate, and skin, hematopoetic tumors of lymphoid and myeloid lineage, tumors of mesenchymal origin, tumors of central peripheral nervous systems, other tumors, including melanoma, seminoma, teratocarcinoma, osteosarcoma, xeroderma pigmentosum, keratoxanthoma, thyroid follicular cancer, and Karposi's sarcoma. Preferably, compounds of Formula II are suited to image prostate cancer and its metastases. Due to comparable mechanisms being part of the cancer cells not only prostate cancer but also lung, including small and non-small cell lung cancer, and breast cancer can be imaged preferably by compounds of Formula II.

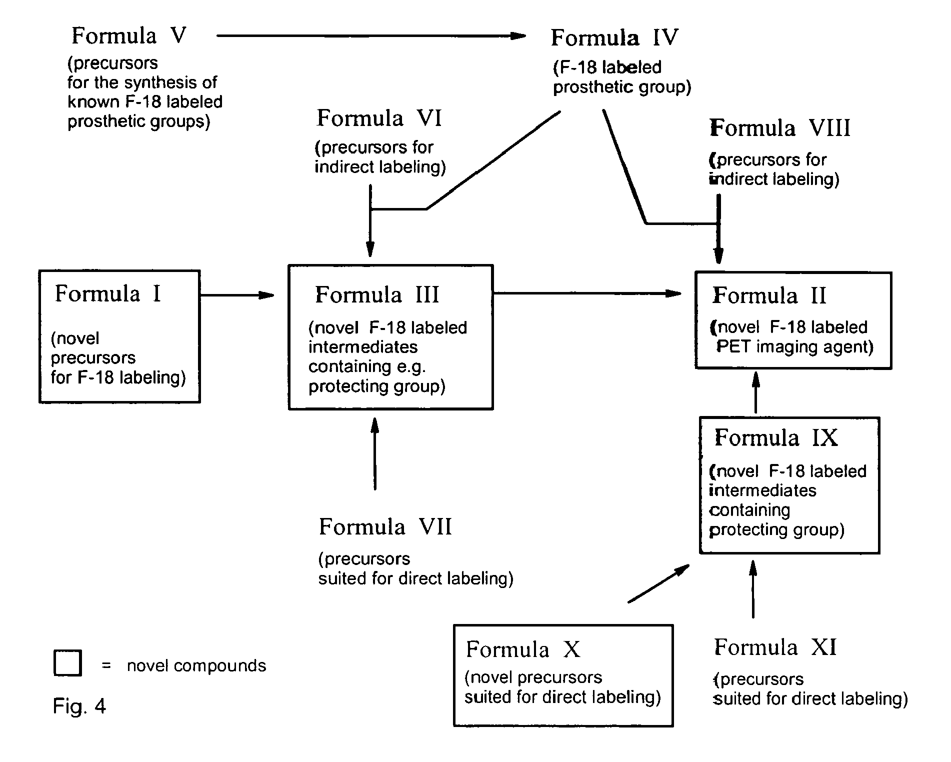

### Detailed Description of the Invention:

*In a **first aspect*** the present invention is directed to compounds of Formula I 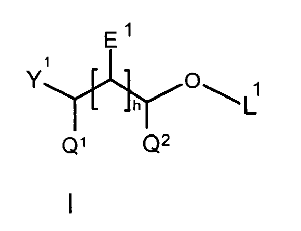 or pharmaceutically acceptable salts of an inorganic or organic acid thereof, hydrates, complexes, esters, amides, solvates or prodrugs thereof,
wherein
**Y¹** is selected from the group comprising
   a) -N(A¹)-(CH₂)ᵤ-Q³ and
   b) -N=C(U¹)(U²);
in a preferred embodiment **Y¹** is selected from the group comprising
   a) -N(A¹)-(CH₂)ᵤ-Q³;
**Q¹, Q²** and **Q³** are selected individually and independently from the group comprising:
   a) hydrogen,
   b) L²-(C₁-C₄)alkyl,
   c) L²-(C₂-C₄)alkenyl and
   d) L²-(C₂-C₄)alkynyl;
in a preferred embodiment **Q¹, Q²** and **Q³** are selected individually and independently from the group comprising
   a) hydrogen and
   b) L²-(C₁-C₃)alkyl;
in a more preferred embodiment **Q¹, Q²** and **Q³** are selected individually and independently from the group comprising
   a) hydrogen and
   b) L²-(C₁-C₂)alkyl;
**A¹** is selected from the group comprising
   a) methyl,
   b) ethyl and
   c) L³;
in a more preferred embodiment **A¹** is selected from the group comprising
   a) methyl and
   b) L³;
**U¹** and **U²** are selected individually and independently from the group comprising
   a) aryl,
   b) *tert*-butyl,
   c) hydrogen,
   d) methylsulfanyl,
   e) halo-aryl,
   f) hydroxyl-aryl,
   g) nitro-aryl,
   h) (C₁-C₄)alkyl-aryl and
   i) ((C₁-C₄)alkyloxy)-aryl;
in a preferred embodiment **U¹** and **U²** are selected individually and independently from the group comprising
   a) aryl,
   b) methoxy-aryl and
   c) methyl-aryl;
in a more preferred embodiment **U¹** and **U²** are selected individually and independently from the group comprising
   a) phenyl,
   b) methylphenyl and
   c) methoxyphenyl;
**E¹** is selected from a group comprising
   a) hydrogen,
   b) L²,
   c) L²-(C₁-C₄)alkyl,
   d) L²-(C₂-C₄)alkenyl and
   e) L²-(C₂-C₄)alkynyl;
in a preferred embodiment **E¹** is selected from the group comprising
   a) hydrogen,
   b) L² and
   c) L²-(C₁-C₃)alkyl;
in a more preferred embodiment **E¹** is selected from the group comprising
   a) hydrogen,
   b) L² and
   c) L²-(C₁-C₂)alkyl;
**L¹** is selected from the group comprising
   a) hydrogen and
   b) **protecting group** which is known or obvious to someone skilled in the art, which is chosen from but not limited to a class of protecting groups namely ethers, benzyl ethers, silyl ethers, esters, carbonates, sulfonates, acetals, ketals, ortho esters and boronates and which is chosen from but not limited to those described in the textbook Greene and Wuts, Protecting groups in Organic Synthesis, third edition, page 17-245, included herewith by reference;
in a preferred embodiment **L¹** is selected from the group comprising
   a) hydrogen,
   b) *tert*-butyl,
   c) triphenylmethyl,
   d) 2-tetrahydropyranyl,
   e) (1-methoxy)cyclohexyloxy,
   f) acetyl,
   g) methoxymethyl and
   h) α-naphtyldiphenylmethyl;
in a more preferred embodiment **L¹** is selected from the group comprising
   a) hydrogen,
   b) *tert*-butyl,
   c) 2-tetrahydropyranyl,
   d) methoxy-methyl and
   e) (1-methoxy)cyclohexyloxy;
**L²** is a **leaving group** - but not chloro, bromo, iodo, mesyloxy or tosyloxy - which is known or obvious to someone skilled in the art and which is taken from but not limited to those described or named in Synthesis (1982), p. 85-125, table 2 (p. 86; (the last entry of this table 2 needs to be corrected: "n-C₄F₉S(O)₂-O- nonaflat" instead of "n-C₄H₉S(O)₂-O-nonaflat"), Carey and Sundberg, Organische Synthese, (1995), page 279-281, table 5.8; or Netscher, Recent Res. Dev. Org. Chem., 2003, 7, 71-83, scheme 1, 2, 10 and 15.
in a preferred embodiment **L²** is selected from the group comprising
   a) (4-bromo-phenyl)sulfonyloxy,
   b) (4-nitro-phenyl)sulfonyloxy,
   c) (2-nitro-phenyl)sulfonyloxy,
   d) (4-isopropyl-phenyl)sulfonyloxy,
   e) (2,4,6-tri-isopropyl-phenyl)sulfonyloxy,
   f) (2,4,6-trimethyl-phenyl)sulfonyloxy,
   g) (4-*tert*butyl-phenyl)sulfonyloxy and
   h) (4-methoxy-phenyl)sulfonyloxy;
in a more preferred embodiment **L²** is selected from the group comprising
   a) (4-bromo-phenyl)sulfonyloxy,
   b) (4-nitro-phenyl)sulfonyloxy,
   c) (4-isopropyl-phenyl)sulfonyloxy and
   d) (2,4,6-tri-isopropyl-phenyl)sulfonyloxy;

**L³** is a protecting group.
The **protecting group** is known or obvious to someone skilled in the art, which is chosen from but not limited to a class of protecting groups namely carbamates, amides, imides, *N-*alkyl amines, *N*-aryl amines, imines, enamines, boranes, N-P protecting groups, N-sulfenyl, N-sulfonyl and N-silyl, and which is chosen from but not limited to those described in the textbook Greene and Wuts, Protecting groups in Organic Synthesis, third edition, page 494-653, included herewith by reference;
in a preferred embodiment **L³** is selected from the group comprising
   a) (tert-butoxy)-carbonyl,
   b) triphenylmethyl,
   c) ((para-methoxy)phenyl-diphenyl)methyl,
   d) (1-adamantyloxy)carbonyl,
   e) (diphenylmethoxy)carbonyl,
   f) (cinnamoyloxy)carbonyl,
   g) (cyclobutyloxy)carbonyl,
   h) ((1-methyl)cyclobutyloxy)carbonyl,
   i) ((1-methyl-1-phenyl)ethyloxy)carbonyl,
   j) ((1-methyl-1-(4-biphenylyl))ethyloxy)carbonyl,
   k) (vinyloxy)carbonyl,
   l) formly,
   m) pivaloyloxymethyl and
   n) diphenylphosphinyl;
in a more preferred embodiment **L³** is selected from the group comprising
   a) (tert-butoxy)-carbonyl,
   b) triphenylmethyl,
   c) (diphenylmethoxy)carbonyl,
   d) ((1-methyl-1-phenyl)ethoxy)carbonyl and
   e) formyl;
in a even more preferred embodiment **L³** is selected from the group comprising
   a) (tert-butoxy)-carbonyl,
   b) triphenylmethyl, and
   c) formyl;
**u** is an integer of from 0 to 4; preferably **u** is an integer of from 0 to 2 and more preferably **u** is an integer of from 0 to 1;
   **h** is an integer of from 0 to 3; preferably **h** is an integer of from 0 to I and more preferably **h** is 0;

In a preferred embodiment compounds of Formula I contain exactly one L².

In a preferred embodiment, compound of formula I is a pharmaceutical acceptable hydrates, complexes, esters, amides, solvates or prodrugs thereof.

Preferred compounds are

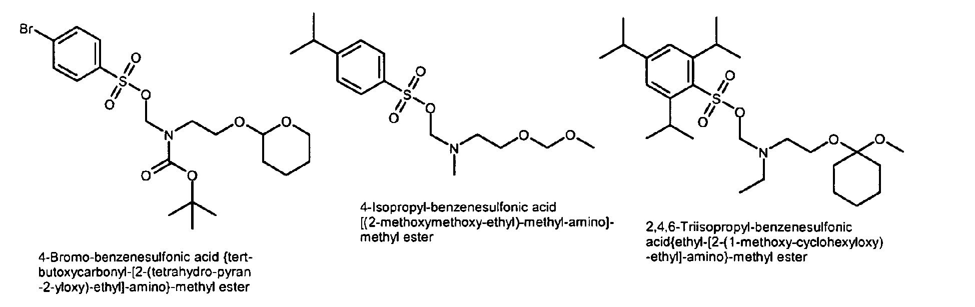

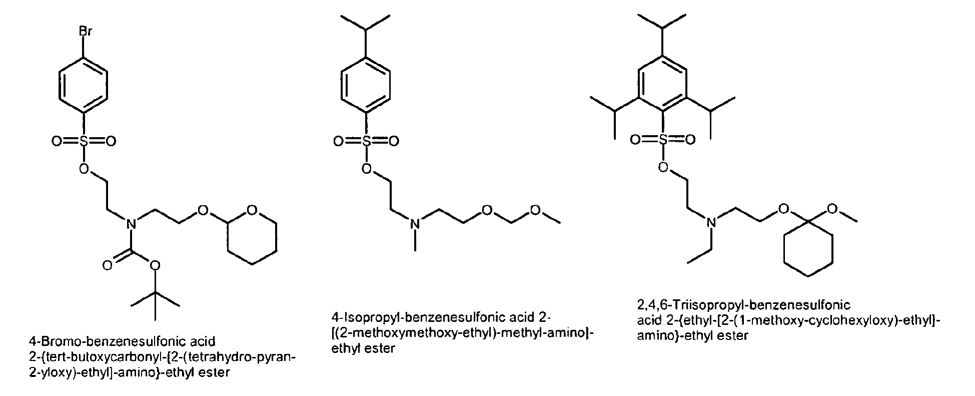

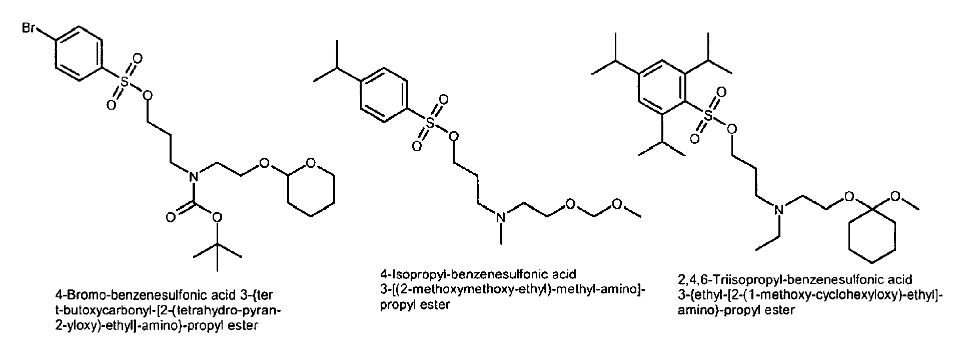

*In a **second aspect*** the present invention is directed to compounds of Formula II 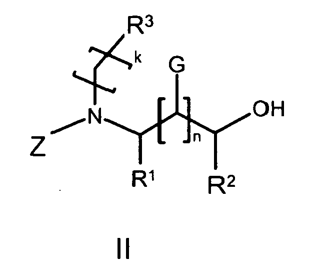 or pharmaceutically acceptable salts of an inorganic or organic acid thereof, hydrates, complexes, esters, amides, solvates or prodrugs thereof,
wherein
**R¹, R²** and **R³** are selected individually and independently from a group comprising:
   a) hydrogen and
   b) [F-18]-fluoro-(C₁-C₄)alkyl;
   c) [F-18]-fluoro-(C₂-C₄)alkenyl and
   d) [F-18]-fluoro-(C₂-C₄)alkynyl;
in a preferred embodiment **R¹, R²** and **R³** are selected individually and independently from a group comprising:
   a) hydrogen and
   b) [F-18]-fluoro-(C₁-C₄)alkyl, more preferably (C₁-C₄)alkyl is a (C₁-C₃)alkyl or (C₁-C₂)alkyl.
**G** is selected from the group comprising:
   a) hydrogen,
   b) [F-18]-fluoro,
   c) [F-18]-fluoro-(C₁-C₄)alkyl,
   d) [F-18]-fluoro-(C₂-C₄)alkenyl and
   e) [F-18]-fluoro-(C₂-C₄)alkynyl;
in a preferred embodiment **G** is selected from a group comprising:
   a) hydrogen,
   b) [F-18]-fluoro and
   c) [F-18]-fluoro-(C₁-C₄)alkyl, more preferably (C₁-C₄)alkyl is a (C₁-C₃)alkyl or (C₁-C₂)alkyl.
**Z** is selected from the group comprising
   a) hydrogen,
   b) (C₁-C₄)alkyl
in a preferred embodiment Z is selected from the group comprising
   a) hydrogen,
   b) methyl, and
   c) ethyl;
**k** is an integer of from 0 to 4; preferably **k** is an integer of from 0 to 2 and more preferably **k** is an integer of from 0 to 1;
   **n** is an integer of from 0 to 3; preferably **n** is an integer of from 0 to 1 and more preferably **n** is 0 ;

In a preferred embodiment compounds of Formula II contain exactly one [F-18]-fluoro atom.

In a preferred embodiment compound of formula II is a pharmaceutical acceptable hydrates, complexes, esters, amides, solvates or prodrugs thereof.

Preferred compounds are

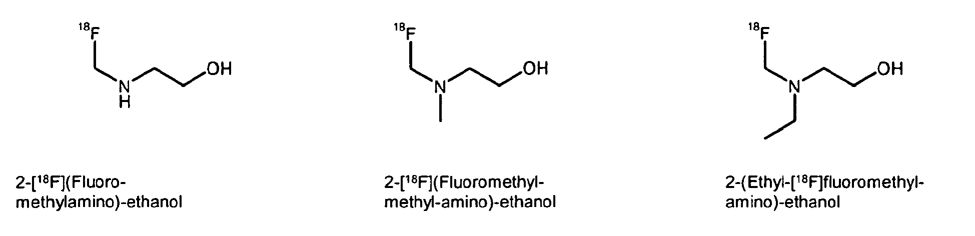

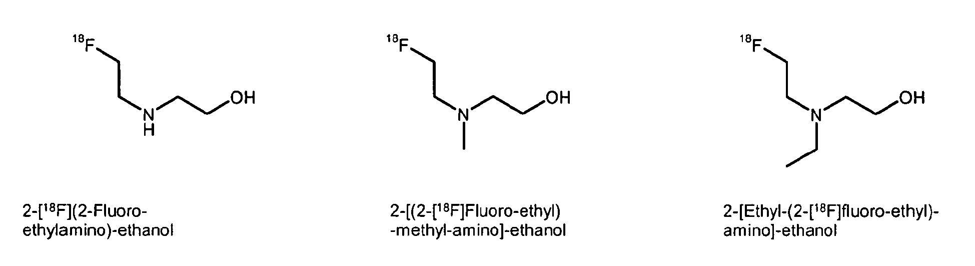

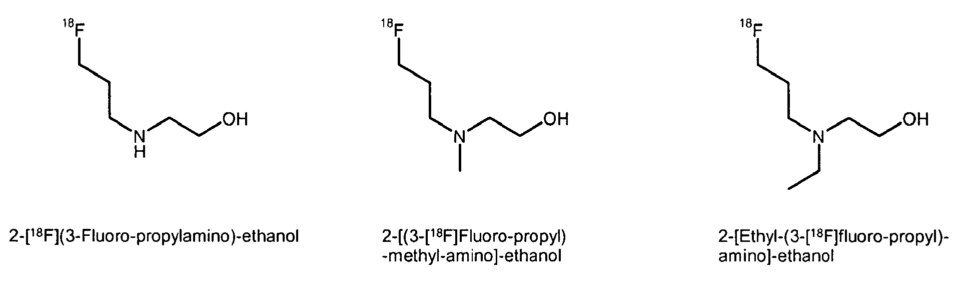

*In a **third aspect*** of the invention compounds according to Formula II are provided as medicament or pharmaceutical.
The invention relates also to the use of compound of Formula II for the manufacture of medicament or pharmaceutical for treatment.
In a more preferred embodiment the use concerns the treatment of cancer. Cancer includes but is not limited to: carcinoma such as bladder, breast, colon, kidney, liver, lung, including small cell lung cancer, esophagus, gall-bladder, ovary, pancreas, stomach, cervix, thyroid, prostate, and skin, hematopoetic tumors of lymphoid and myeloid lineage, tumors of mesenchymal origin, tumors of central peripheral nervous systems, other tumors, including melanoma, seminoma, teratocarcinoma, osteosarcoma, xeroderma pigmentosum, keratoxanthoma, thyroid follicular cancer, and Karposi's sarcoma.
More preferably, the cancer is selected from lung cancer, including small cell lung cancer, prostate cancer, breast cancer, ovary cancer or colon cancer.

The present invention is also directed to a method for treatment comprising the step of introducing into a patient a suitable quantity of a labeled compound of Formula II.

*In a **fourth aspect*** of the invention compounds according to Formula II are provided as diagnostic imaging agent or imaging agent, preferably as imaging agent for PET applications.
The invention relates also to the use of compound of Formula II for the manufacture of imaging agent.
In a more preferred embodiment the use concerns the imaging of cancer. Cancer includes but is not limited to: carcinoma such as bladder, breast, colon, kidney, liver, lung, including small cell lung cancer, esophagus, gall-bladder, ovary, pancreas, stomach, cervix, thyroid, prostate, and skin, hematopoetic tumors of lymphoid and myeloid lineage, tumors of mesenchymal origin, tumors of central peripheral nervous systems, other tumors, including melanoma, seminoma, teratocarcinoma, osteosarcoma, xeroderma pigmentosum, keratoxanthoma, thyroid follicular cancer, and Karposi's sarcoma.

More preferably, the cancer is selected from lung cancer, including small cell lung cancer, prostate cancer, breast cancer, ovary cancer or colon cancer.

The present invention is also directed to a method for imaging comprising the step of introducing into a patient a detectable quantity of a labeled compound of Formula II and imaging said patient.

*In a **fifth aspect*** of the invention, pharmaceutical composition comprising compounds according to Formula I or pharmaceutically acceptable salts of an inorganic or organic acid thereof, hydrates, complexes, esters, amides, solvates or prodrugs thereof is provided. Preferably the pharmaceutical composition comprises a physiologically acceptable carrier, diluent, adjuvant or excipient.

In a preferred embodiment pharmaceutical composition comprises compound of formula I that is a pharmaceutical acceptable hydrates, complexes, esters, amides, solvates or prodrugs thereof.

*ln a **sixth aspect*** of the invention, radiopharmaceutical composition comprising compounds according to Formula II or pharmaceutically acceptable salts of an inorganic or organic acid thereof, hydrates, complexes, esters, amides, solvates or prodrugs thereof is provided.

In a preferred embodiment compound of formula II is a pharmaceutical acceptable hydrates, complexes, esters, amides, solvates or prodrugs thereof.

Preferably the pharmaceutical composition comprises a physiologically acceptable carrier, diluent, adjuvant or excipient.

The radioactively labeled compounds according to Formula II provided by the invention may be administered intravenously in any pharmaceutically acceptable carrier, e.g. conventional medium such as an aqueous saline medium, or in blood plasma medium, as a pharmaceutical composition for intravenous injection. Such medium may also contain conventional pharmaceutical materials such as, for example, pharmaceutically acceptable salts to adjust the osmotic pressure, buffers, preservatives and the like. Among the preferred media are normal saline solution and plasma.
Suitable pharmaceutical acceptable carriers are known to someone skilled in the art. In this regard reference can be made to e.g. Remington's Practice of Pharmacy, 13th ed. and in J. of. Pharmaceutical Science & Technology, Vol. 52, No. 5, Sept-Oct., p. 238-311, included herein by reference.

The concentration of the compound according to Formula II and the pharmaceutically acceptable carrier, for example, in an aqueous medium, varies with the particular field of use. A sufficient amount is present in the pharmaceutically acceptable carrier when satisfactory visualization of the imaging target (e.g. a tumor) is achievable.

*In a **seventh aspect*** of the invention is directed to compounds of Formula III, 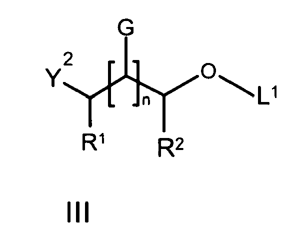 or pharmaceutically acceptable salts of an inorganic or organic acid thereof, hydrates, complexes, esters, amides, solvates or prodrugs thereof,
wherein
**Y²** is selected from the group comprising
   a) -N(A²)-(CH₂)ₖ-Q³ and
   b) -N=C(U³)(U⁴);
in a preferred embodiment **Y²** is selected from the group comprising
   a) -N(A²)-(CH₂)ₖ-Q³;
**R¹, R²** and **R³** are selected individually and independently from a group comprising:
   a) hydrogen,
   b) [F-18]-fluoro-(C₁-C₄)alkyl,
   c) [F-18]-fluoro-(C₂-C₄)alkenyl and
   d) [F-18]-fluoro-(C₂-C₄)alkynyl;
in a preferred embodiment **R¹, R²** and **R³** are selected individually and independently from a group comprising:
   a) hydrogen and
   b) [F-18]-fluoro-(C₁-C₄)alkyl, more preferably (C₁-C₄)alkyl is a (C₁-C₃)alkyl or (C₁-C₂)alkyl;
**G** is selected from the group comprising:
   a) hydrogen,
   b) [F-18]-fluoro,
   c) [F-18]-fluoro-(C₁-C₄)alkyl,
   d) [F-18]-fluoro-(C₂-C₄)alkenyl and
   e) [F-18]-fluoro-(C₂-C₄)alkynyl;
in a preferred embodiment **G** is selected from a group comprising:
   a) hydrogen,
   b) [F-18]-fluoro and
   c) [F-18]-fluoro-(C₁-C₄)alkyl, more preferably (C₁-C₄)alkyl is a (C₁-C₃)alkyl or (C₁-C₂)alkyl;
**A²** is selected from the group comprising
   a) methyl,
   b) ethyl and
   c) L³;
in a more preferred embodiment **A²** is selected from the group comprising
   a) methyl and
   b) L³;
**U³** and **U⁴** are selected individually and independently from the group comprising
   a) aryl,
   b) *tert*-butyl,
   c) hydrogen,
   d) methylsulfanyl,
   e) halo-aryl,
   f) hydroxyl-aryl,
   g) nitro-aryl,
   h) (C₁-C₄)alkyl-aryl and
   i) ((C₁-C₄)alkyloxy)-aryl;
in a preferred embodiment **U³** and **U⁴** are selected individually and independently from the group comprising
   a) aryl,
   b) ((C₁-C₄)alkyloxy)-aryl, and
   c) (C₁-C₄)alkyl-aryl;
in a more preferred embodiment aryl is phenyl;
in a more preferred embodiment ((C₁-C₄)alkyloxy)-aryl is methoxy-aryl wherein aryl is preferably a phenyl;
in a more preferred embodiment (C₁-C₄)alkyl-aryl is methyl-aryl wherein aryl is preferably a phenyl;
   **L¹** is as defined above;
   **L³** is as defined above;
   **k** is an integer of from 0 to 4; preferably **k** is an integer of from 0 to 2 and more preferably **k** is an integer of from 0 to 1;
      **n** is an integer of from 0 to 3; preferably **n** is an integer of from 0 to 1 and more preferably **n** is 0 ;

In a preferred embodiment compounds of Formula III contain exactly one [F-18]-fluoro atom.

In a preferred embodiment compound of formula III is a pharmaceutical acceptable hydrates, complexes, esters, amides, solvates or prodrugs thereof.

Preferred compounds are

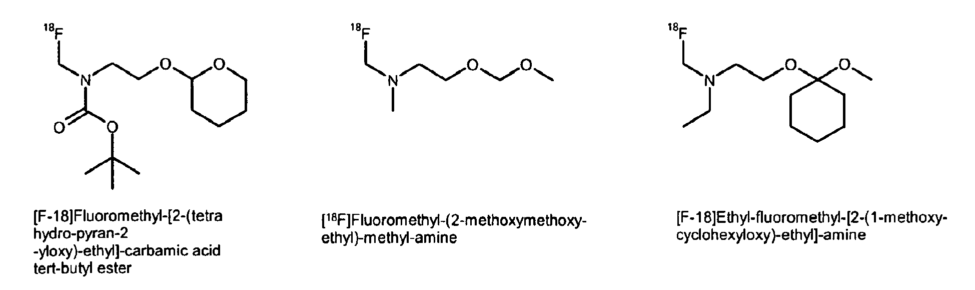

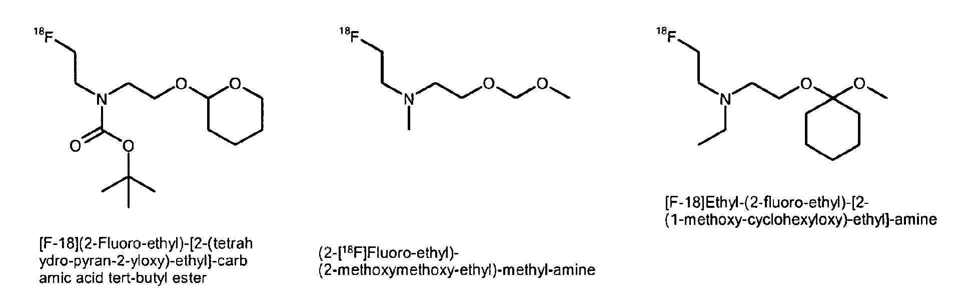

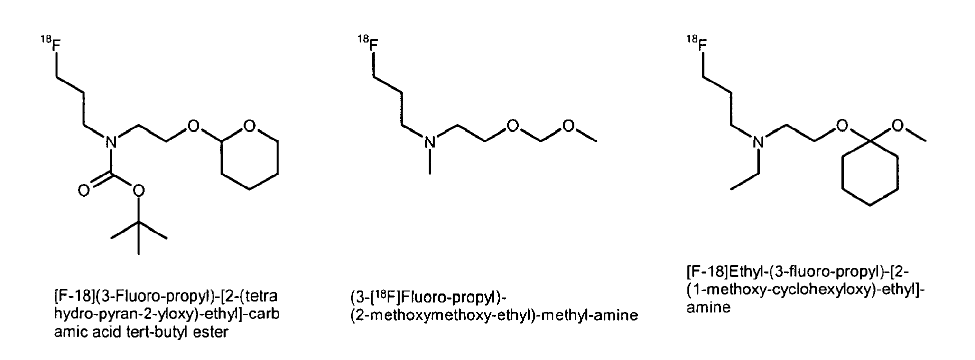

*In a **eighth aspect*** of the invention is directed to a method for obtaining compounds of Formula III, wherein k, n, R¹, R², R³, G, A², L¹, L³, U³ and U⁴ are as defined above. This includes in particular all preferred embodiments mentioned above.
Surprisingly three methods have been identified for obtaining compounds of Formula III.

The first method comprises a straight forward fluoro labeling reaction i.e. one-step method from compounds of Formula I for obtaining compound of formula III.
The radiolabeling method for obtaining compound of formula III comprises the step of
- Reacting a compound of formula I with a fluorinating agent for obtaining a compound of formula III.

In a preferred embodiment, the fluorination agent is a fluorine radioactive isotope derivative. More preferably the fluorine radioactive isotope derivative is a ¹⁸F derivative. More preferably, the ¹⁸F radioactive isotope derivative is 4,7,13,16,21,24-Hexaoxa-1,10-diazabicyclo[8.8.8]-hexacosane K18F (crownether salt Kryptofix K18F), K¹⁸F, H¹⁸F, KH¹⁸F₂, Cs¹⁸F, Na¹⁸F or tetraalkylammonium salt of ¹⁸F (e.g.[F-18] tetrabutylammonium fluoride).
More preferably, the fluorination agent is K¹⁸F, H¹⁸F, or KH¹⁸F₂ , most preferably K¹⁸F (¹⁸F fluoride anion).

The second method comprises the step of substitution of the nitrogen functionality of compounds of Formula VI using a fluorine labeled compound of Formula IV as substituent for obtaining compound of formula III.
The radiolabeling method for obtaining compound of formula III comprises the steps of
- Obtaining a compound of formula IV from a compound of formula V, and
- Reacting a compound of formula IV with a compound of formula VI.

The compound of Formula IV is 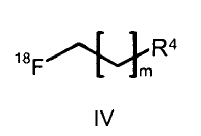 or pharmaceutically acceptable salts of an inorganic or organic acid thereof, hydrates, complexes, esters, amides, solvates or prodrugs thereof,
wherein
**m** is an integer from 0 to 4, preferably **m** is an integer of from 0 to 2 and more preferably **m** is an integer of from 0 to 1, and
**R⁴** is a leaving group.

**The leaving group** is known or obvious to someone skilled in the art and which is taken from but not limited to those described or named in Synthesis (1982), p. 85-125, table 2 (p. 86; (the last entry of this table 2 needs to be corrected: "n-C₄F₉S(O)₂-O- nonaflat" instead of "n-C₄H₉S(O)₂-O- nonaflat"), Carey and Sundberg, Organische Synthese, (1995), page 279-281, table 5.8; or Netscher, Recent Res. Dev. Org. Chem., 2003, 7, 71-83, scheme 1, 2, 10 and 15.

In a more preferred embodiment **R⁴** is selected from the group comprising:
a) iodo,
b) bromo,
c) chloro,
d) mesyloxy,
e) tosyloxy,
f) trifluormethylsulfonyloxy and
g) nonafluorobutylsulfonyloxy;

The compounf of Formula V is 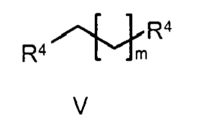 or pharmaceutically acceptable salts of an inorganic or organic acid thereof, hydrates, complexes, esters, amides, solvates or prodrugs thereof,
wherein
**R⁴** is defined as above, and
**m** is an integer from 0 to 4, preferably **m** is an integer of from 0 to 2 and more preferably **m** is an integer of from 0 to 1.

The compound of Formula VI is 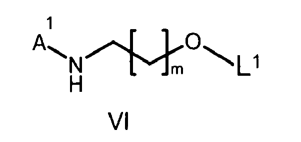 or pharmaceutically acceptable salts of an inorganic or organic acid thereof, hydrates, complexes, esters, amides, solvates or prodrugs thereof,
wherein
**A¹** is selected from the group comprising:
   a) methyl,
   b) ethyl and
   c) L³ (protecting group);
**L³** is defined as above,
**L¹** is defined as above, and
**m** is an integer from 0 to 4, preferably **m** is an integer of from 0 to 2 and more preferably **m** is an integer of from 0 to 1.

The method is described in scheme 3.

The third method comprises a straight forward radioisotope labeling reaction i.e. one-step method from compounds of Formula VII using [F-18] fluoride anions.
The radiolabeling method for obtaining compound of formula III comprises the steps of
- Reacting a compound of formula VII with fluorination agent.

The fluorination agent is defined as above.

The compound of Formula VII is 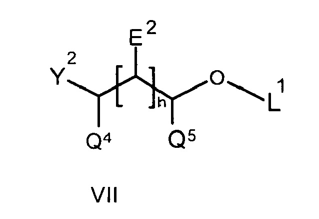 or pharmaceutically acceptable salts of an inorganic or organic acid thereof, hydrates, complexes, esters, amides, solvates or prodrugs thereof,
wherein
**Y²** is selected from the group comprising
   a) -N(A³)-(CH₂)ᵤ-Q⁶ and
   b) -N=C(U⁵)(U⁶),
In a more preferred embodiment **Y²** is selected from the group comprising:
   a) -N(A³)-(CH₂)ᵤ-Q⁶,
**Q⁴, Q⁵** and **Q⁶** are selected individually and independently from the group comprising:
   a) hydrogen and
   b) L⁴-(C₁-C₄)alkyl;
   c) L⁴-(C₂-C₄)alkenyl and
   d) L⁴-(C₂-C₄)alkynyl;
In a preferred embodiment **Q⁴, Q⁵** and **Q⁶** are selected individually and independently from the group comprising
   a) hydrogen and
   b) L⁴-(C₁-C₄)alkyl, more preferably (C₁-C₄)alkyl is a (C₁-C₃)alkyl or (C₁-C₂)alkyl;
**A³** is selected from the group comprising
   a) methyl,
   b) ethyl and
   c) L³ (protecting group);
In a more preferred embodiment **A³** is selected from the group comprising
   a) methyl and
   b) L³ (protection group);
**U⁵** and **U⁶** are selected individually and independently from the group comprising
   a) aryl,
   b) *tert*-butyl,
   c) hydrogen,
   d) methylsulfanyl,
   e) halo-aryl,
   f) hydroxyl-aryl
   g) nitro-aryl,
   h) (C₁-C₄)alkyl-aryl and
   i) ((C₁-C₄)alkyloxy)-aryl;
in a more preferred embodiment aryl is phenyl;
in a more preferred embodiment ((C₁-C₄)alkyloxy)-aryl is methoxy-aryl wherein aryl is preferably a phenyl;
in a more preferred embodiment (C₁-C₄)alkyl-aryl is methyl-aryl wherein aryl is preferably a phenyl;
**E²** is selected from a group comprising
   a) hydrogen,
   b) L⁴,
   c) L⁴-(C₁-C₄)alkyl,
   d) L⁴-(C₂-C₄)alkenyl and
   e) L⁴-(C₂-C₄)alkynyl;
in a preferred embodiment **E²** is selected from the group comprising
   a) hydrogen,
   b) L⁴ and
   c) L⁴-(C₁-C₄)alkyl, more preferably (C₁-C₄)alkyl is a (C₁-C₃)alkyl or (C₁-C₂)alkyl;
**L¹** is defined as above;
**L⁴** is a leaving group and is selected from the group comprising
   a) chloro,
   b) bromo,
   c) iodo,
   d) mesyloxy and
   e) tosyloxy;
in a preferred embodiment **L⁴** is selected from the group
   a) bromo,
   b) mesyloxy and
   c) tosyloxy;
**u** is an integer of from 0 to 4; preferably **u** is an integer of from 0 to 2 and more preferably **u** is an integer of from 0 to 1;
**h** is an integer of from 0 to 3; preferably **h** is an integer of from 0 to 1 and more preferably **h** is 0 ;

In a preferred embodiment compounds of Formula VII contain exactly one leaving group.

In a preferred embodiment compound of formula VII is a pharmaceutical acceptable hydrates, complexes, esters, amides, solvates or prodrugs thereof.

*In a **ninth aspect*** of the invention is directed to novel compounds of Formula IX 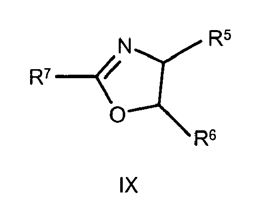 or pharmaceutically acceptable salts of an inorganic or organic acid thereof, hydrates, complexes, esters, amides, solvates or prodrugs thereof,
wherein
**R⁵** and **R⁶** are selected individually and independently from a group comprising:
   a) hydrogen and
   b) [F-18]-fluoro-(C₁-C₄)alkyl,
   c) [F-18]-fluoro-(C₂-C₄)alkenyl and
   d) [F-18]-fluoro-(C₂-C₄)alkynyl;
in a preferred embodiment **R⁵**and **R⁶** are selected individually and independently from a group comprising:
   a) hydrogen and
   b) [F-18]-fluoro-(C₁-C₄)alkyl, more preferably (C₁-C₄)alkyl is a (C₁-C₃)alkyl or (C₁-C₂)alkyl;
**R⁷** is selected from a group comprising:
   a) (C₁-C₆)alkyl,
   b) (C₁-C₆)alkenyl,
   c) (C₁-C₆)alkynyl,
   d) aryl and
   e) ar-(C₁-C₆)alkyl;
in a more preferred embodiment, (C₁-C₆)alkyl of **R⁷** is selected from the group (C₁-C₃)alkyl, more preferably methyl or ethyl;
   in a more preferred embodiment, aryl of **R⁷** is selected from phenyl;

In a more preferred embodiment compounds of Formula IX contain exactly one [F-18]-fluoro atom.

*In a **tenth aspect*** of the invention is directed to novel compounds of Formula X 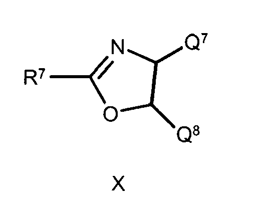 or pharmaceutically acceptable salts of an inorganic or organic acid thereof, hydrates, complexes, esters, amides, solvates or prodrugs thereof, wherein
**Q⁷** and **Q⁸** are selected individually and independently from the group comprising:
   a) hydrogen,
   b) L²-(C₁-C₄)alkyl,
   c) L²-(C₂-C₄)alkenyl and
   d) L²-(C₂-C₄)alkynyl;

In a preferred embodiment **Q⁷** and **Q⁸** are selected individually and independently from the group comprising
a) hydrogen and
b) L²-(C₁-C₄)alkyl, more preferably (C₁-C₄)alkyl is a (C₁-C₃)alkyl or (C₁-C₂)alkyl;
wherein **R⁷** and **L²** are defined as above.

In a preferred embodiment, compounds of Formula X contain exactly one L².

*In an **eleventh aspect*** of the invention is directed to a method for obtaining compounds of Formula IX, wherein **R⁵, R⁶** and **R⁷** are as defined above.

Surprisingly two methods have been identified to obtain compounds of Formula IX.

The first method consists of the reaction of compounds of Formula IX with fluorination agent. The radiolabeling method for obtaining compound of formula IX comprises the step of
- Reacting a compound of formula XI with fluorination agent.

Compounds of formula IX is as defined above.
The fluorination agent is defined as above.

The compound of Formula XI is 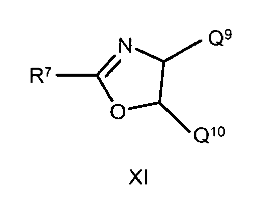 or pharmaceutically acceptable salts of an inorganic or organic acid thereof, hydrates, complexes, esters, amides, solvates or prodrugs thereof, wherein
**Q⁹** and **Q¹⁰** are selected individually and independently from the group comprising
   a) hydrogen;
   b) L⁴-(C₁-C₄)alkyl;
   c) L⁴-(C₂-C₄)alkenyl and
   d) L⁴-(C₂-C₄)alkynyl;
in a preferred embodiment **Q⁹** and **Q¹⁰** are selected individually and independently from the group comprising
   a) hydrogen and
   b) L⁴-(C₁-C₄)alkyl, more preferably (C₁-C₄)alkyl is a (C₁-C₃)alkyl or (C₁-C₂)alkyl;
   wherein **R⁷** and **L⁴** is defined as above.

In a preferred embodiment compounds of Formula XI contain exactly one L⁴.

The second method is the reaction of compounds of Formula X, whereas **Q⁷, Q⁸, L²** and **R⁷** is defined as above, with fluorination agent..
The radiolabeling method for obtaining compound of formula IX comprises the step of
- Reacting a compound of formula X with fluorination agent.

Compounds of formula IX and X is as defined above.
The fluorination agent is defined as above.

*In a **twelfth aspect*** of the invention is directed to a method for obtaining compounds of Formula II, wherein **R¹, R², R³, G** and **Z** are as defined above.

Surprisingly three methods have been identified for obtaining compounds of Formula II.

The first method for obtaining compounds of Formula II comprises the step of a straight forward deprotection of a compounds of Formula III which are generated from compounds of Formula I or VII.
The radiolabeling method for obtaining compound of formula II comprises the steps of
- Radiolabelling of a compound of formula I or VII with a fluorination agent for obtaining a compound of formula III, and
- Deprotecting compound of formula III.

Compounds of formula I, II, III and VII are as defined above.
The fluorination agent is defined as above.

The second method for obtaining compounds of Formula II comprises the step of substitution reaction of a fluorine labeled compound of Formula IV, wherein **R⁴** is defined as above, with the nitrogen functionality of a compound of Formula VIII.
The radiolabeling method for obtaining compound of formula II comprises the steps of
- Radiolabeling of compound of formula V with a radioactive fluorination agent for obtaining a compound of formula IV, and
- Substituing compound of formula IV with compound of Formula VIII.

Compounds of formula II, IV, and V are as defined above.
The fluorination agent is defined as above.

The compound of Formula VIII is 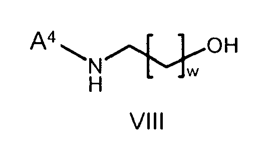 or pharmaceutically acceptable salts of an inorganic or organic acid thereof, hydrates, complexes, esters, amides, solvates or prodrugs thereof,
wherein **A⁴** is
a) hydrogen,
b) methyl or
c) ethyl,

**w** is an integer of from 0 to 3; preferably **w** is an integer of from 0 to 1 and more preferably **w** is 0 ;

In a preferred embodiment, the fluorination agent is a fluorine radioactive isotope derivative. More preferably the fluorine radioactive isotope derivative is a ¹⁸F derivative. More preferably, the ¹⁸F derivative is 4,7,13,16,21,24-Hexaoxa-1,10-diazabicyclo[8.8.8]-hexacosane K18F (crownether salt Kryptofix K18F), K¹⁸F, H¹⁸F, KH¹⁸F₂, Cs¹⁸F, Na¹⁸F or tetraalkylammonium salt of ¹⁸F (e.g.[F-18] tetrabutylammonium fluoride). More preferably, the fluorination agent is K¹⁸F, H¹⁸F, or KH¹⁸F₂ , most preferably K¹⁸F (¹⁸F fluoride anion).

The third method comprises a straight forward step of deprotection from compounds of Formula IX, wherein **R⁵, R⁶** and **R⁷** which are generated from compounds of Formula X or XI.
The radiolabeling method for obtaining compound of formula II comprises the steps of
- Radiolabelling of a compound of formula X or XI with a fluorination agent for obtaining a compound of formula IX, and
- Deprotecting compound of formula II.

Compounds of formula II, IX, X and XI are as defined above.

*In a **thirteenth aspect*** of the invention compounds according to Formula III or IX are provided as medicament or pharmaceutical.
The invention relates also to the use of compound of Formula III or IX for the manufacture of medicament or pharmaceutical for treatment.
In a more preferred embodiment the use concerns the treatment of cancer. Cancer includes but is not limited to: carcinoma such as bladder, breast, colon, kidney, liver, lung, including small cell lung cancer, esophagus, gall-bladder, ovary, pancreas, stomach, cervix, thyroid, prostate, and skin, hematopoetic tumors of lymphoid and myeloid lineage, tumors of mesenchymal origin, tumors of central peripheral nervous systems, other tumors, including melanoma, seminoma, teratocarcinoma, osteosarcoma, xeroderma pigmentosum, keratoxanthoma, thyroid follicular cancer, and Karposi's sarcoma.
More preferably, the cancer is selected from lung cancer, including small cell lung cancer, prostate cancer, breast cancer, ovary cancer or colon cancer.

The present invention is also directed to a method for treatment comprising the step of introducing into a patient a suitable quantity of a labeled compound of Formula III or IX.

*In a **fourteenth aspect*** of the invention, compounds according to Formula III or IX are provided as diagnostic imaging agent or imaging agent, preferably as imaging agent for PET applications.
The invention relates also to the use of compound of Formula III or IX for the manufacture of imaging agent.

In a more preferred embodiment the use concerns the imaging of cancer. Cancer includes but is not limited to: carcinoma such as bladder, breast, colon, kidney, liver, lung, including small cell lung cancer, esophagus, gall-bladder, ovary, pancreas, stomach, cervix, thyroid, prostate, and skin, hematopoetic tumors of lymphoid and myeloid lineage, tumors of mesenchymal origin, tumors of central peripheral nervous systems, other tumors, including melanoma, seminoma, teratocarcinoma, osteosarcoma, xeroderma pigmentosum, keratoxanthoma, thyroid follicular cancer, and Karposi's sarcoma.

More preferably, the cancer is selected from lung cancer, including small cell lung cancer, prostate cancer, breast cancer, ovary cancer or colon cancer.

The present invention is also directed to a method for imaging comprising the step of introducing into a patient a detectable quantity of a labeled compound of Formula III or IX and imaging said patient.

*In a* ***fifteenth aspect*,** the invention is directed to a kit comprising a vial comprising a predetermined quantity of a compound having any one of the following general chemical Formulae or mixture thereof
a) compounds of Formula I;
b) compounds of Formula V and VI;
c) compounds of Formula V and VIII;
d) compounds of Formula VII;
e) compounds of Formula X or
f) compounds of Formula XI.

Further, according to this aspect of the present invention the kit comprises a compound having general chemical Formula as disclosed above along with an acceptable carrier, diluent, excipient or adjuvant or mixture thereof

*In a* ***sixteenth aspect*,** the invention is directed to compound, method, pharmaceutical composition and kit for any fluorine isotope. It means that even the invention is describing ¹⁸F labelled compound, ¹⁸F radiolabeling method, and ¹⁸F pharmaceutical composition the invention encompasses compound of the invention comprising any fluorine isotope (radioactive and non radioactive isotope), for for fluoro labelling and for

### Definitions

The term "alkyl" refers to a linear or branched chain monovalent or divalent radical consisting of solely carbon and hydrogen, containing no unsaturation and having the specified number of carbons, such as methyl (C₁), ethyl (C₂), n-propyl (C₃), 1-methlyethyl ((C₃) iso-propyl), n-butyl (C₄), n-pentyl (C₅) and the like. More preferably alkyl is C₁-C₄ alkyl.

The term "Alkenyl" is similarly defined as for alkyl, but contain at least one carbon-carbon double bond, respectively. More preferably alkenyl is C₂-C₄ alkyl.

The term "Alkynyl" is similarly defined as for alkyl, but contain at least one carbon-carbon triple bond, respectively. More preferably alkynyl is C₂-C₄ alkyl.

The term "aryl" as employed herein by itself or as part of another group refers to monocyclic or bicyclic aromatic groups containing from 6 to 12 carbons in the ring portion, preferably 6-10 carbons in the ring portion, such as phenyl, naphthyl or tetrahydronaphthyl.

As used hereinafter in the description of the invention and in the claims, the term "heterocycloalkyl", by itself or as part of another group, refers to groups having 5 to 14 ring atoms of a cycloalkyl; and containing carbon atoms and 1, 2, 3 or 4 oxygen, nitrogen or sulfur heteroatoms.. More preferably heterocycloalkyl is C₃-C₁₀ heterocycloalkyl, C₅-C₈ heterocycloalkyl or C₅-C₁₄ heterocycloalkyl.

The term halogen or halo refers to Cl, Br, F or I.

The term "alkyloxy" or "alkoxy" refers to alkyl groups respectively linked by an oxygen atom, with the alkyl being as defined above.

The term "ar-alkyl" as employed herein by itself or as part of another group refers to monocyclic or bicyclic aromatic groups containing from 6 to 12 carbons in the ring portion, preferably 6-10 carbons in the ring portion, such as phenyl, naphthyl or tetrahydronaphthyl which is substituted by or linked via a linear or branched chain monovalent or divalent radical consisting of solely carbon and hydrogen, containing no unsaturation and having the specified number of C₁-C₆ carbons, such as methyl (C₁), ethyl (C₂), n-propyl (C₃), 1-methlyethyl ((C₃) iso-propyl), n-butyl (C₄), n-pentyl (C₅), n-(hexyl) (C6) and the like.

As used hereinafter in the description of the invention and in the claims, the term "fluorine isotope" (F) refers to all isotopes of the fluorine atomic element. Fluorine isotope (F) is selected from radioactive or non-radioactive isotope. The radioactive fluorine isotope is selected from ¹⁸F . The non-radioactive "cold" fluorine isotope is selected from ¹⁹F.

As used hereinafter in the description of the invention and in the claims, the term "prodrug" means any covalently bonded compound, which releases the active parent pharmaceutical according to formula II.
The term "prodrug"as used throughout this text means the pharmacologically acceptable derivatives such as esters, amides and phosphates, such that the resulting in vivo biotransformation product of the derivative is the active drug as defined in the compounds of formula (I). The reference by Goodman and Gilman (The Pharmaco- logical Basis of Therapeutics, 8 ed, McGraw-HiM, Int. Ed. 1992,"Biotransformation of Drugs", p 13-15) describing prodrugs generally is hereby incorporated. Prodrugs of a compound of the present invention are prepared by modifying functional groups present in the compound in such a way that the modifications are cleaved, either in routine manipulation or in vivo, to the parent compound. Prodrugs of the compounds of the present invention include those compounds wherein for instance a hydroxy group, such as the hydroxy group on the asymmetric carbon atom, or an amino group is bonded to any group that, when the prodrug is administered to a patient, cleaves to form a free hydroxyl or free amino, respectively.
Typical examples of prodrugs are described for instance in WO 99/33795, WO 99/33815, WO 99/33793 and WO 99/33792 all incorporated herein by reference.
Prodrugs are characterized by excellent aqueous solubility, increased bioavailability and are readily metabolized into the active inhibitors in vivo.

As used hereinafter in the description of the invention and in the claims, the terms "salts of inorganic or organic acids", "inorganic acid" and "organic acid" refer to mineral acids, including, but not being limited to: acids such as carbonic, nitric, phosphoric, hydrochloric, perchloric or sulphuric acid or the acidic salts thereof such as potassium hydrogen sulphate, or to appropriate organic acids which include, but are not limited to: acids such as aliphatic, cycloaliphatic, aromatic, araliphatic, heterocyclic, carboxylic and sulphonic acids, examples of which are formic, acetic, trifluoracetic, propionic, succinic, glycolic, gluconic, lactic, malic, fumaric, pyruvic, benzoic, anthranilic, mesylic, fumaric, salicylic, phenylacetic, mandelic, embonic, methansulfonic, ethanesulfonic, benzenesulfonic, phantothenic, toluenesulfonic, trifluormethansulfonic and sulfanilic acid, respectively.

As used hereinafter in the description of the invention and in the claims, the term "pharmaceutically acceptable salt" relates to salts of inorganic and organic acids, such as mineral acids, including, but not limited to, acids such as carbonic, nitric or sulfuric acid, or organic acids, including, but not limited to, acids such as aliphatic, cycloaliphatic, aromatic, araliphatic, heterocyclic, carboxylic and sulphonic acids, examples of which are formic, acetic, trifluoroacetic, propionic, succinic, glycolic, gluconic, lactic, malic, fumaric, pyruvic, benzoic, anthranilic, mesylic, salicylic, phenylacetic, mandelic, embonic, methansulfonic, ethanesulfonic, benzenesulfonic, phantothenic, toluenesulfonic and sulfanilic acid.

### Examples

An example for the synthesis of compounds of Formula I towards compounds of Formula III and II is depicted in scheme 1: compound **6** (e.g. Acta Chim. Acad. Sci. Hung.; 2; 1952; 153, 159) and 1-methoxy-hex-1-ene (Aldrich) are converted (Tetrahedron (1995), 51, 11, 3339-3344) to the acetal **8**.

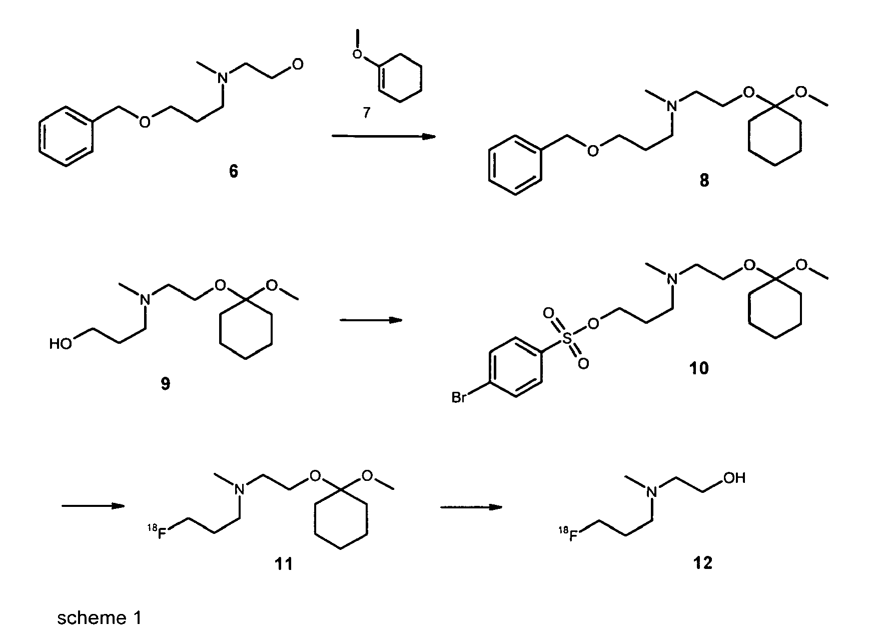

The benzyl ether **8** is cleaved by heterogeneous hydrogenation with hydrogen gas and palladium on coal (J. Am. Chem. Soc., 93, 1746 (1971)). A typical solvent for such a reaction is methanol or iso-propanol. Alcohol **9** is converted to the corresponding brosylate **10.** This reaction is carried out using (p-bromo-benzene)-sulfonic acid and small amounts of 4-N,N-dimethylamino-pyridine (DMAP) in dichloromethane containing base (pyridine or trimethylamine) (J. Org. Chem. (1996), 61, 2, 587-597). Compound **10** is converted to the F-18 labeled compound **11** using F-18 fluoride, potassium carbonate and "kryptofix" (2.2.2 crown ether, Aldrich). This and other conditions for such radiofluorination are known to experts (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50). For example DMF, DMSO and alcohols can be used as solvent and caesium carbonate or potassium oxalate as base. The radiofluorination can be carried out in a "hot-cell" and/or by use of a module (eview: Krasikowa, Synthesis Modules and Automation in F-18 labeling (2006), in: Schubiger P.A., Friebe M., Lehmann L., (eds), PET-Chemistry - The Driving Force in Molecular Imaging. Springer, Berlin Heidelberg, pp. 289-316) which allows an automated or semi-automated synthesis. The protecting group of compounds **11** is deprotected towards compound **12** with acid, whereas mineral acids, like hydrogen chloride, are preferred. The conversion from compound **11** towards compound **12** is carried out at elevatated temperature.

But depending on the strength of acid and reaction times also lower temperatures, e.g. room temperature is possible. Compounds of Formula I are more suite for F-18 labeling than compounds of Formula VII towards the synthesis of compounds of formuala III. The leaving group of compounds of Formula I are optimal to obtain a compound of Formula III and Formula II because they allow radio-fluorination reactions with superior radiochemical yield and/or specific activity.
Nevertheless, it is possible to synthesize compounds of Formula III conveniently from compounds of Formula VII. Scheme 2 depicts an example for the synthesis of a compound of Formula III and Formula II from a compound of Formula VII.

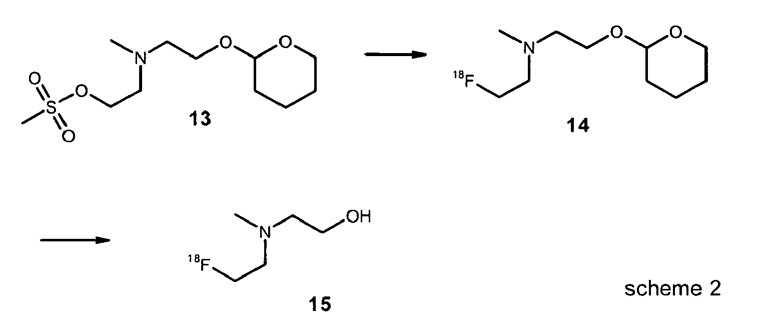

Compound **13** is radio-fluorinated with [F-18]fluoride, potassium carbonate and crown ether (kryptofix) in acetonitrile, dimethyl formamide or dimethyl sulfoxide to obtain compound 14. This radio-fluorination can carried by a single operator by "hand" or on a module (see above) by automated or semi-automated methods Krasikowa 2006)). Compound **14** is deprotected using acid, preferably mineral acid, more preferably hydrogen chloride, perchloric acid or sulfuric acid. After deprotection of compound **14** compound **15** is obtained which is typically purified using cartouches or HPLC-columns.

Compounds of Formula III (and subsequently to compounds of Formula II) can also be obtained by reacting compounds of Formula VI with [F-18] labeled compounds of Formula IV (prosthetic group) which are generated from compounds of Formula V - an example is depicted in scheme 3:

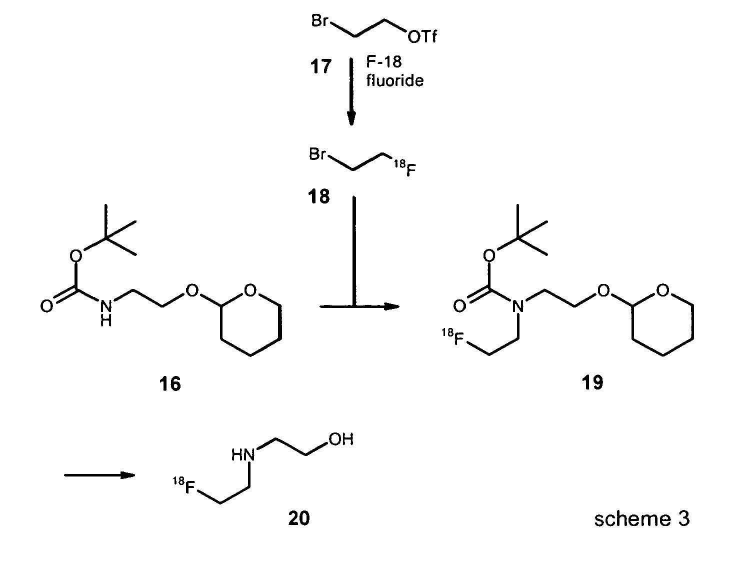

The nitrogen of the *N*-Boc-protected amine **16** (Eur J Org Chem (2006), 6, 1476-1482) is deprotonated with sodium hydride and subsequently alkylated with [F-18]2-fluoro-ethyl bromide (**18**) which is generated from 2-bromo ethyl triflate (Bioorg. Med. Chem.; 11; 12; 2003; 2519 - 2528). The protection groups of compound **19** are cleaved with acid, preferably with mineral acid, more preferably with hydrogen chloride, perchloric acid or sulfuric acid, to obtain [F-18]-labeled amino-alcohol **20.** The conversion from compound **19** towards compound **20** is carried out at elevated temperature. But depending on the strength of acid and reaction times also lower temperatures, e.g. room temperature is possible.

An example for the synthesis of compounds of Formula II from compounds of Formula VIII is shown in scheme 4. An excess of amine **21** (Aldrich) is alkylated with compound **18** (Bioorg. Med. Chem.; 11; 12; 2003; 2519 - 2528) being generated from compound **17** with potassium carbonate as base in dimethyl formamide. The purification of the desired compound **22** is carried out with preparative HPLC methods.

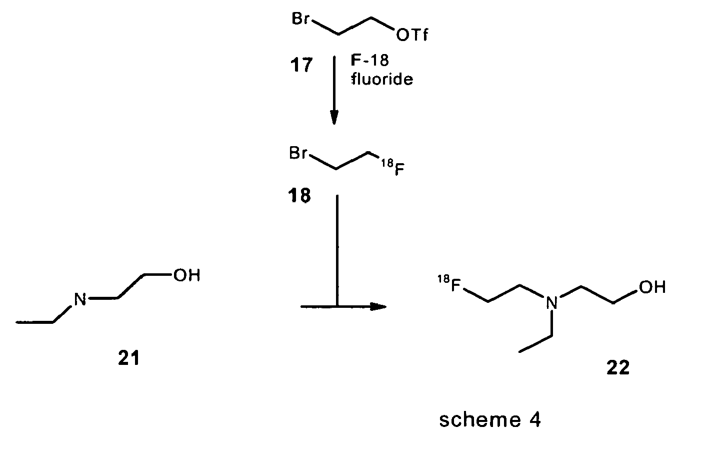

An example for the synthesis of compounds of Formula II from compounds of Formula IX which are generated from compounds of Formula XI is depicted in scheme 5.

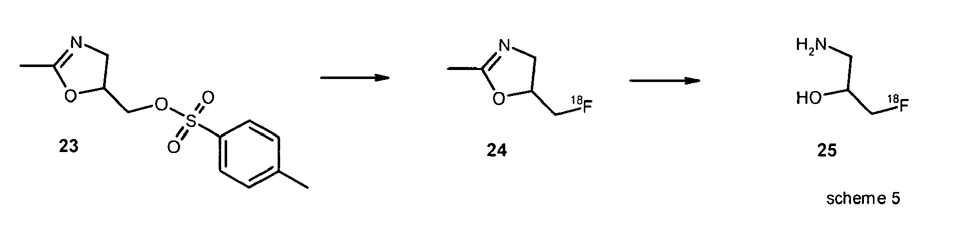

Compound **23** (J. Org. Chem.; 64; 26; 1999; 9337 - 9347) is radiofluorinated to obtain compound **24**. The reagents, solvents and conditions which can be used for this radio fluorination are common and well-known to skilled person in the field (see e.g. J. Fluorine Chem. 27 (1985) 117-191). Compound **24** is subsequently deprotected by use of acid, preferably with mineral acid, more preferably with hydrogen chloride, perchloric acid or sulfuric acid. The resulted compound **25** is purified by cartouches and/or HPLC techniques.

An example for the synthesis of compounds of Formula II from compounds of formula IX which are generated from compounds of formula X is depicted in scheme 6.

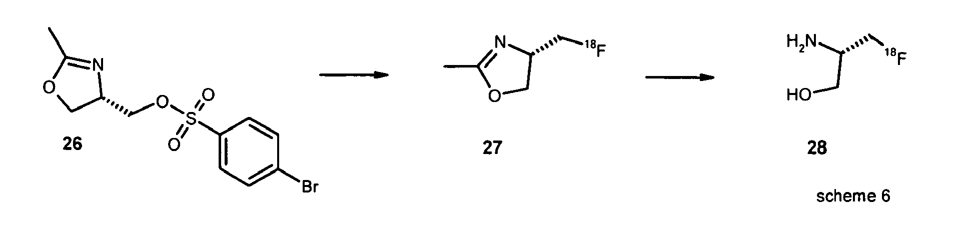

Compound **26** is synthesized from compound ((R)-2-methyl-4,5-dihydro-oxazol-4-yl)-methanol (J. Org. Chem.; 44; 1979; 2250-2256) by use of (4-bromo-benzene)sulfonyl chloride. The sulfonate leaving group is substituted in a S_{N}2 reaction by [F-18]-fluoride to obtain compound 27. The cleavage of the protecting group is carried out by acidic hydrolysis using preferably mineral acid, e.g. hydrogen chloric acid. The desired compound **28** is obtained and is purified by HPLC methods.

### Experimental Part

### General procedures:

### A: Fluorination with non-radioactive [F-19] fluoride

To a solution of 1 mmol starting material in 2 ml acetonitril 63.5 mg (1.1 mmol) potassium fluoride and 414mg g (1.1 eq.) kryptofix are added. The reaction mixture is heated by microwave (130° C, 15 min) and cooled to room temperature again. The reaction mixture is diluted with 30 ml diethyl ether and 30 ml water. The organic phase is separated. The aqueous phase is extracted three times with 30 ml diethyl ether. The combined organic phases are washed with brine and dried with magnesium sulfate. The solvent is evaporated and the residue is purified by column chromatography with ethyl acetate-hexane gradient.

### B: Fluorination with radioactive [F-18] fluoride

To a Wheaton vial (5ml) charged with 2.5 mg Kryptofix (2.2.2Kryptand) in 0.75 ml acetonitrile and 0.5 mg potassium carbonate and the fluorine containing water (0.5 - 2.5 GBq, 200-300µl) is added. The solvent is removed by heating at 120°C for 10 mins under a stream of nitrogen. Anhydrous MeCN (1 ml) is added and evaporated as before. This step is repeated again. A solution of starting material (2 mg) in 0.70 ml anhydrous MeCN is added. After heating at 130°C for 30 min. The mixture is cooled to room temperature and treated with 1 ml 5N aqueous hydrogen chloride solution. The reaction mixture is heated to 100°C for 10 min.

The crude reaction mixture is analyzed using analytical HPLC: ACE3-C18 50 mm x 4,6 mm; solvent gradient: start 5%acetonitril - 95%acetonitril in water in 7 min., flow: 2ml/min. The desired F-18 labeled product is confirmed by co-injection with the non-radioactive F-19 fluoro-standard on the analytical HPLC. If not deprotected (see general procedure C) the crude product (50-400 MBq) is purified by preparative HPLC column: ACE5-C18-HL 250 mm x 10 mm; solvent 75% acetonitril-35% water, isocratic 20 min., flow: 3 ml/min. The desired product is obtained (15-200 MBq) as reconfirmed by co-injection with the non-radioactive F-19 fluoro standard on the analytical HPLC.

### C: Cleavage of protecting groups of [F-18] labeled derivative

The crude [F-18]labeled product is treated with 2ml 4N HCl solution. The reaction mixture is heated for 6-7 minutes to 110°C. The reaction mixture is cooled. The desired F-18 labeled product is confirmed by co-injection with the non-radioactive F-19 fluoro-standard on the analytical HPLC. The crude product (50-400 MBq) is purified by preparative HPLC column: ACE5-C18-HL 250 mm x 10 mm; solvent 75% acetonitril-35% water, isocratic 20 min., flow: 3 ml/min. The desired product is obtained (15-200 MBq) as reconfirmed by co-injection with the non-radioactive F-19 fluoro standard on the analytical HPLC.

### D: Alkylation of NH-carbamate with [F-18] labeled prosthetic group

To a suspension of 1ml dry tetrahydrofuran (THF) and 7.7 mmol sodium hydride - which has been washed with hexane - 7 mmol starting material in 1 ml THF is added dropwisely. The reaction mixture is stirred for 20 min. The prepared [F-18]-fluoro-alkyl bromide (100-500 GBq; known from literature) in tetrahydrofuran is dropped into the suspension. The reaction is heated to 50°C for 20 min. The vigorously reaction mixture is cooled to room temperature. The crude reaction mixture is analyzed using analytical HPLC: ACE3-C18 50 mm x 4,6 mm; solvent gradient: start 5%acetonitril - 95%acetonitril in water in 7 min., flow: 2ml/min. The desired F-18 labeled product is confirmed by co-injection with the non-radioactive F-19 fluoro-standard on the analytical HPLC.

### E: Cleavage of protecting group after the alkylation of NH-carbamate with [F-18] labeled prosthetic group

The reaction mixture is dropewisly diluted with 1ml acetonitril and 2,5 ml aqueous hydrogenchloride (6N). The reaction mixture is heated to 100°C for 10 min. The crude reaction mixture is analyzed using analytical HPLC: ACE3-C18 50 mm x 4,6 mm; solvent gradient: start 5%acetonitril - 95%acetonitril in water in 7 min., flow: 2ml/min. The desired F-18 labeled product is confirmed by co-injection with the non-radioactive F-19 fluoro-standard on the analytical HPLC. The crude product (50-400 MBq) is purified by preparative HPLC column: ACE5-C18-HL 250 mm x 10 mm; solvent 75% acetonitril-35% water, isocratic 20 min., flow: 3 ml/min. The desired product is obtained (15-200 MBq) as reconfirmed by co-injection with the non-radioactive F-19 fluoro standard on the analytical HPLC.

### F: Alkylation of NH-carbamate

To a stirred suspension of 20ml dry tetrahydrofuran (THF) and 11 mmol sodium hydride - which has been washed with hexane - 10 mmol starting material in 5 ml THF is added dropwisely at 0°C. The reaction mixture is stirred for 20 min. 15 mmol alkylation agent diluted in 5ml tetrahydrofuran is added dropwisely to the stirred suspension. The reaction mixture is stirred for 16-10 hours. The reaction mixture is poured onto a vigerously stirred mixture of ice-water and diethyl ether. The organic phase is separated. The aqueous phase is extracted three times with 30 ml diethyl ether. The combined organic phases are washed with brine and dried with magnesium sulfate. The solvent is evaporated and the residue is purified by column chromatography with ethyl acetate-hexane gradient.

### G: Alkylation of NH-amine with [F-18] labeled prosthetic group

To a solution 2 mg secondary amine and 3 mg potassium carbonate in 0,7 ml dimethyl formamide was added [F-18]fluoro-alkylating agent (ca. 200-1000 MBq) in dimethyl formamide prepared from literature protocol. The reaction mixture is heated to 110°C for 20 min. The reaction mixture is cooled to room temperature. The desired F-18 labeled product is confirmed by co-injection with the non-radioactive F-19 fluoro-standard on the analytical HPLC. The crude product (ca. 50-400 MBq) is purified by preparative HPLC column: ACE5-C18-HL 250 mm x 10 mm; solvent 75% acetonitril-35% water, isocratic 20 min., flow: 3 ml/min. The desired product is obtained (ca. 15-200 MBq) as reconfirmed by co-injection with the non-radioactive F-19 fluoro standard on the analytical HPLC.

### H: Alkylation of NH-amine (secondary amine)

To a stirred solution of 20 mmol starting material and 4.15 g (30 mmol) potassium carbonate in 60 ml dimethyl formamide was added 25 mmol alkylating agent. The reaction mixture was heated by microwave to 110°C for 15 min. The solvent of the reaction mixture is evaporated.

Water (80 ml) and diethylether or dichloromethane/isopropanol mixture (1:10 - 80 ml) are added. The organic phase is separated. The aqueous phase is extracted three times with 30 ml diethyl ether. The combined organic phases are washed with water (twice ca. 50ml), brine and dried with magnesium sulfate. The solvent is evaporated and the residue is purified by column chromatography with ethyl acetate-hexane gradient.

### I: Conversion of alcohol to corresponding O-substituted sulfonate (version 1)

To a solution of 5 mmol starting material and 1,03 g (8 mmol) diisopropyl ethyl amine in 15 ml dichloromethane was added (6 mmol) mesyl chloride in 1 ml dichloromethane dropwise at -10°C. The stirred reaction mixture was warmed over a period of 4,5 h to room temperature and diluted with dichloromethane. The organic phase was washed with saturated sodium hydrogen carbonate solution, water and brine. The organic phase was dried with magnesium sulfate. The crude product was purified by silica column chromatography (ethyl acetate-hexane gradient).

### K: Conversion of alcohol to corresponding O-substituted sulfonate (version 2)

To a solution of 3 mmol starting material in 5 ml dichloromethane and 5 ml pyridine was added (3.3 mmol) aryl sulfonyl chloride in 1 ml dichloromethane dropwisely at -10°C. The stirred reaction mixture was warmed over a period of 4,5 h to room temperature and diluted with dichloromethane. The organic phase was washed with 0.5N sulfuric acid (three times), saturated sodium hydrogen carbonate solution, water and brine. The organic phase was dried with magnesium sulfate. The crude product was purified by silica column chromatography (ethyl acetate-hexane gradient).

### L: Heterogenous hydrogenation

To a stirred solution of ca. 20-50 mg palladium on coal (10%) in 15 ml isopropanol 8 mmol starting material were added in 5 ml iso-propanol. The reaction mixture is stirred at hydrogen atmosphere for 16-20 hours. The reaction mixture is filtered; and the solvent is evaporated. The residue is purified by column chromatography with ethyl acetate-hexane gradient.

### M: Deprotection of acid labile protecting group (Version 1)

A solution of 0.5 mmol starting material in wet trifluoro acetic acid-dichloromethane mixture (1:1) was stirred for 4-7 hours. The reaction mixture is evaporated. The residue is solved in dichloromethane and the solution is evaporated again. The last step is repeated three times.

The residue is purified by column chromatography (dichloromethane - pentane gradient, amino phase).

### N: Deprotection of acid labile protecting group (Version 2)

(According to J. Am. Chem. Soc., 6644, 92, (1970))

To a stirred solution of 0.5 mmol starting material in 1ml ethanol is added 1ml of 3N aqueous hydrogen chloride at 0°C. The solution is stirred for 16h at room temperature. The reaction is treated with NaOH aq. (4N) until pH = 9.5. Ethanol is evaporated. Water (10ml) and dichloromethan-isopropanol (10 ml; 10:1) are added. The organic phase is separated. The aqueous phase is extracted three times with 10 ml dichloromethan-isopropanol (10:1). The combined organic phases are washed with brine and dried with magnesium sulfate. The solvent is evaporated and the residue is purified by column chromatography with ether-pentane gradient or by preparative HPLC methods.

### O: Protection of alcohols as (2-tetrahydropyranyl) (THP) ethers or as (1-methoxy-1-cyclohexyl)methyl ether.

To a stirred solution of 8 mmol starting material and 200 mg (0.8 mmol) PPTS in 35 ml dichloromethane is added 840 mg (10 mmol) 2,3-dihydropyran or 1,12 g (10 mmol) 1-methoxy-cyclohex-1-ene in 10 ml dichloromethane dropwisely at 0°C. The reaction mixture is stirred for 16-20 hours. The reaction mixture is diluted with 30 ml dichloromethane and 60 ml water. The organic phase is separated. The aqueous phase is extracted three times with diethyl ether. The combined organic phases are washed with brine and dried with magnesium sulfate. The solvent is evaporated and the residue is either used as crude procuct or purified by column chromatography with ethyl acetate-hexane gradient.

### Example 1

### a) Synthesis of (3-Benzyloxy-propyl)-[2-(1-methoxy-cyclohexyloxy)-ethyl]-methyl-amine (1a)

The desired compound (**1a**) was obtained according to general procedure O in 85% yield (6.8 mmol, 2.27 g).
MS-ESI: 336 (M⁺ +1, 100).

| Elementary analysis: | | | |
|---|---|---|---|
| Calculated: | C 71.60% | H 9.91% | N 4.17% |
| Determined: | C 71.57% | H 9.89% | N 4.16% |

### b) Synthesis of 3-{[2-(1-Methoxy-cyclohexyloxy)-ethyl]-methyl-amino}-propan-1-ol (1b).

The desired compound **1b** is obtained from compound **1a** according to the general procedure L in 87% yield (4.35 mmol, 1.07g).
MS-ESI: 246 (M⁺ +1, 100).

| Elementary analysis: | | | |
|---|---|---|---|
| Calculated: | C 63.64% | H 11.09% | N 5.71% |
| Determined: | C 63.62% | H 11.10% | N 5.71% |

### c) Synthesis of 4-Bromo-benzenesulfonic acid 3-{[2-(1-methoxy-cyclohexyloxy)-ethyl]-methyl-amino}propyl ester (1c)

The desired compound **1c** is obtained from **1b** according to the general procedure K in 76% yield (2.28 mmol, 1.06g).
MS-ESI: 465 (M⁺ +1, 100).

| Elementary analysis: | | | |
|---|---|---|---|
| Calculated: | C 49.14% | H 6.51% | N 3.02% |
| Determined: | C 49.15% | H 6.52% | N 3.01% |

### d) Synthesis of(3-Fluoro-propyl)-[2-(1-methoxy-cyclohexyloxy)-ethyl]-methyl-amine (1d)

The desired compound **1d** is obtained from compound **1c** according to the general procedure A in 69% yield (0.69 mmol, 170 mg).
MS-ESI: 248 (M⁺ +1, 100).

| Elementary analysis: | | | |
|---|---|---|---|
| Calculated: | C 63.13% | H 10.59% | N 5.66% |
| Determined: | C 63.15% | H 10.58% | N 5.65% |

### e) Synthesis of [F-18](3-Fluoro-propyl)-[2-(1-methoxy-cyclohexyloxy)-ethyl]-methyl-amine (1e)

The desired compound **1e** was obtained from compound **1c** according to general procedure B .

### f) Synthesis of 2-[(3-Fluoro-propyl)-methyl-amino]-ethanol (1f)

The desired compound **1f** was obtained from compound **1d** according to general procedure M in 56% yield (35 mg, 0.26 mmol).
MS-ESI: 136 (M⁺ +1, 89).

| Elementary analysis: | | | |
|---|---|---|---|
| Calculated: | C 53.31% | H 10.44% | N 10.36% |
| Determined: | C 53.30% | H 10.44% | N 10.36% |

### g) [F-18]2-[(3-Fluoro-propyl)-methyl-amino]-ethanol (1g)

The desired compound 1h was obtained from compound 1f according to general procedure C in 34% overall radiochemical yield.

### Example 2

### a) Synthesis of 2-{Methyl-[2-(tetrahydro-pyran-2-yloxy)-ethyl]-amino}-ethanol (2a)

### [(2-Hydroxy-ethyl)-methyl-amino]-ethanol (Aldrich)

MS-ESI: 204 (M⁺ +1, 100).

| Elementary analysis: | | | |
|---|---|---|---|
| Calculated: | C 59.09% | H 10.41% | N 6.89% |
| Determined: | C 59.10% | H 10.41% | N 6.90% |

### b) Methanesulfonic acid 2-{methyl-[2-(tetrahydro-pyran-2-yloxy)-ethyl]-amino}-ethyl ester (2b)

The desired compound **2b** was obtained from **2a** according to the general procedure I in 74% yield (3.7 mmol, 1.04 g).
MS-ESI: 282 (M⁺ +1, 100).

| Elementary analysis: | | | |
|---|---|---|---|
| Calculated: | C 46.96% | H 8.24% | N 4.98% |
| Determined: | C 46.98% | H 8.24% | N 4.99% |

### c) (2-Fluoro-ethyl)-methyl-[2-(tetrahydro-pyran-2-yloxy)-ethyl]-amine (2c)

The desired compound **2c** was obtained from **2b** according to the general procedure A in 48% yield (0.48 mmol, 98 mg).
MS-ESI: 206 (M⁺ +1, 100).

| Elementary analysis: | | | |
|---|---|---|---|
| Calculated: | C 58.51% | H 9.82% | N 6.82% |
| Determined: | C 58.48% | H 9.83% | N 6.80% |

### d) 2-[(2-Fluoro-ethyl)-methyl-amino]-ethanol (2d)

The desired compound **2d** was obtained from **2c** according to the general procedure M in 61% yield (0.31 mmol, 38 mg).
MS-ESI: 122 (M⁺ +1, 83).

| Elementary analysis: | | | |
|---|---|---|---|
| Calculated: | C 49.57% | H 9.98% | N 11.56% |
| Determined: | C 49.56% | H 9.99% | N 11.54% |

### e) [F-18](2-Fluoro-ethyl)-methyl-[2-(tetrahydro-pyran-2-yloxy)-ethyl]-amine (2e)

The desired compound **2e** was obtained from **2b** according to the general procedure B.

### f) [F-18]2-[(2-Fluoro-ethyl)-methyl-amino]-ethanol (2f)

The desired compound **2f** was obtained from compound **2e** according to the general procedure C in 33% overall radiochemical yield.

### Example 3

### a) Synthesis of (2-Fluoro-ethyl)-[2-(tetrahydro-pyran-2-yloxy)-ethyl]-carbamic acid tert-butyl ester (3a)

The desired compound 3a from [2-(Tetrahydro-pyran-2-yloxy)-ethyl]-carbamic acid tert-butyl ester (Eur J Org Chem (2006), 6, 1476-1482) was obtained according to general procedure F in 78% yield (7.80 mmol, 2.27g).
MS-ESI: 292 (M⁺ +1, 100).

| Elementary analysis: | | | |
|---|---|---|---|
| Calculated: | C 57.71% | H 8.99% | N 4.81% |
| Determined: | C 57.73% | H 8.98% | N 4.82% |

### b) Synthesis of 2-(2-Fluoro-ethylamino)-ethanol (3b)

The desired compound 3b from 3a was obtained according to general procedure M in 62% yield (0.31 mmol, 33mg).
MS-ESI: 108 (M⁺ +1, 100).

| Elementary analysis: | | | |
|---|---|---|---|
| Calculated: | C 44.85% | H 9.41% | N 13.07% |
| Determined: | C 44.86% | H 9.42% | N 13.06% |

### c) Synthesis of [F-18](2-Fluoro-ethyl)-[2-(tetrahydro-pyran-2-yloxy)-ethyl]-carbamic acid tert-butyl ester (3c)

The desired compound **3c** was obtained from [F-18]fluoro-ethylbromide (Bioorg. Med. Chem.; 11; 12; 2003; 2519 - 2528) and [2-(Tetrahydro-pyran-2-yloxy)-ethyl]-carbamic acid tert-butyl ester (Eur J Org Chem (2006), 6, 1476-1482) according to the general procedure D.

### d) Synthesis of [F-18]2-(2-Fluoro-ethylamino)-ethanol (3d)

The desired compound **3d** was obtained from compound 3c according to the general procedure C in 21 % overall chemical yield.

### Example 4

### a) Synthesis of 2-[Ethyl-(2-fluoro-ethyl)-amino]-ethanol (4a)

The desired compound 4a was obtained from 2-ethylamino-ethanol (Aldrich) according to the general procedure H in 46% yield (9.2 mmol, 1.24 g).
MS-ESI: 136 (M⁺ +1, 100).

| Elementary analysis: | | | |
|---|---|---|---|
| Calculated: | C 53.31% | H 10.44% | N 10.36% |
| Determined: | C 53.32% | H 10.42% | N 10.36% |

### b) [F-18]2-[Ethyl-(2-fluoro-ethyl)-amino]-ethanol (4b)

The desired compound **4b** was obtained from 2-ethylamino-ethanol (Aldrich) and [F-18]flouro-ethyl bromide (Bioorg. Med. Chem.; 11; 12; 2003; 2519 - 2528) according to the general procedure G in 18% overall radiochemical yield.

### Example 5

### a) Synthesis of 5-Fluoromethyl-2-methyl-4,5-dihydro-oxazole (5a)

The desired compound 5a was obtained from toluene-4-sulfonic acid 2-methyl-4,5-dihydro-oxazol-5-ylmethyl ester (J. Org. Chem.; 54; 6; 1989; 1295-1304) according to the general procedure A in 67% yield (0.67 mmol, 78,4 mg).
MS-ESI: 118 (M⁺ +1, 100).

| Elementary analysis: | | | |
|---|---|---|---|
| Calculated: | C 51.28% | H 6.88% | N 11.96% |
| Determined: | C 51.28% | H 6.88% | N 11.97% |

### b) Synthesis of 1-Amino-3-fluoro-propan-2-ol (5b)

The desired compound was obtained from compound **5a** according to the procedure M in 54% yield (0,27 mmol; 25 mg).
MS-ESI: 94 (M⁺ +1, 78).

| Elementary analysis: | | | |
|---|---|---|---|
| Calculated: | C 38.70% | H 8.66% | N 15.04% |
| Determined: | C 38.72% | H 8.65% | N 15.05% |

### c) Synthesis of [F-18]5-Fluoromethyl-2-methyl-4,5-dihydro-oxazole (5c)

The desired compound 5c was obtained from compound toluene-4-sulfonic acid 2-methyl-4,5-dihydro-oxazol-5-ylmethyl ester (J. Org. Chem.; 54; 6; 1989; 1295-1304) according to the general procedure B.

### d) Synthesis of [F-18] 1-Amino-3-fluoro-propan-2-ol (5d)

The desired compound 5d was obtained from compound 5c according to procedure C in 19% overall radiochemical yield.

### Example 6

### a) Synthesis of 4-Bromo-benzenesulfonic acid 2-methyl-4,5-dihydro-oxazol-4-ylmethyl ester (6a)

The desired compound **6a** is obtained from compound ((R)-2-methyl-4,5-dihydro-oxazol-4-yl)-methanol (J. Org. Chem.; 44; 1979; 2250-2256) according to the general procedure K in 84% yield (844 mg, 2.52 mmol)
MS-ESI: 335 (M⁺ +1, 100).

| Elementary analysis: | | | |
|---|---|---|---|
| Calculated: | C 39.54% | H 3.62% | N 4.19% |
| Determined: | C 39.58% | H 3.64% | N 4.17% |

### b) Synthesis of 4-Fluoromethyl-2-methyl-4,5-dihydro-oxazole (6b)

The desired compound **6b** is obtained from compound **6a** according to the general procedure A in 56% yield (66 mg, 0.56 mmol)
MS-ESI: 118 (M⁺ + 1, 100).

| Elementary analysis: | | | |
|---|---|---|---|
| Calculated: | C 51.28% | H 6.88% | N 11.96% |
| Determined: | C 51.30% | H 6.88% | N 11.94% |

### c) Synthesis of 2-Amino-3-fluoro-propan-1-ol (6c)

The desired compound **6c** is obtained from compound **6b** according to the general procedure N in 64% yield (0.32 mmol; 30 mg).

### d) Synthesis of [F-18]4-Fluoromethyl-2-methyl-4,5-dihydro-oxazole (6d)

The desired compound **6d** is obtained from compound **6a** according to the general procedure B.

### e) Synthesis of [F-18]2-Amino-3-fluoro-propan-1-ol (6e)

The desired compound **6e** is obtained from compound **6a** according to the general procedure B in 34% overall radiochemical yield (0.32 mmol; 30 mg).

## Claims

1. A compounds of Formula **I** 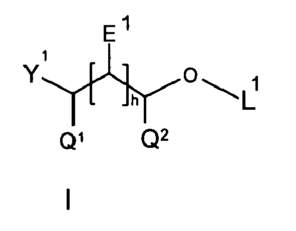 or pharmaceutically acceptable salts of an inorganic or organic acid thereof, hydrates, complexes, esters, amides, solvates or prodrugs thereof,
wherein
**Y¹** is selected from the group comprising
a) -N(A¹)-(CH₂)ᵤ-Q³ and
b) -N=C(U¹)(U²);
**Q¹, Q²** and **Q³** are selected individually and independently from the group comprising:
a) hydrogen,
b) L²-(C₁-C₄)alkyl,
c) L²-(C₂-C₄)alkenyl and
d) L²-(C₂-C₄)alkynyl;
**A¹** is selected from the group comprising
a) methyl,
b) ethyl and
c) L³;
**U¹** and **U²** are selected individually and independently from the group comprising
a) aryl,
b) *tert*-butyl,
c) hydrogen,
d) methylsulfanyl,
e) halo-aryl,
f) hydroxyl-aryl,
g) nitro-aryl,
h) (C₁-C₄)alkyl-aryl and
i) ((C₁-C₄)alkyloxy)-aryl;
**E¹** is selected from a group comprising
a) hydrogen,
b) L²,
c) L²-(C₁-C₄)alkyl,
d) L²-(C₂-C₄)alkenyl and
e) L²-(C₂-C₄)alkynyl;
**L¹** is selected from the group comprising
a) hydrogen and
b) **protecting group**
**L²** is a **leaving group** but not chloro, bromo, iodo, mesyloxy or tosyloxy
**L³** is a protecting group.
u is an integer of from 0 to 4;
h is an integer of from 0 to 3.

2. The compound according to claim 1 wherein **L¹** is selected from the group comprising
i) hydrogen,
j) *tert*-butyl,
k) triphenylmethyl,
l) 2-tetrahydropyranyl,
m) (1-methoxy)cyclohexyloxy,
n) acetyl,
o) methoxymethyl and
p) α-naphtyldiphenylmethyl.

3. The compound according to claims 1 or 2 wherein **L²** is selected from the group comprising
a) (4-bromo-phenyl)sulfonyloxy,
b) (4-nitro-phenyl)sulfonyloxy,
c) (4-isopropyl-phenyl)sulfonyloxy and
d) (2,4,6-tri-isopropyl-phenyl)sulfonyloxy.

4. The compound according to any one of the preceding claims wherein **L³** is selected from the group comprising
a) (tert-butoxy)-carbonyl,
b) triphenylmethyl,
c) ((para-methoxy)phenyl-diphenyl)methyl,
d) (1-adamantyloxy)carbonyl,
e) (diphenylmethoxy)carbonyl,
f) (cinnamoyloxy)carbonyl,
g) (cyclobutyloxy)carbonyl,
h) ((1-methyl)cyclobutyloxy)carbonyl,
i) ((1-methyl-1-phenyl)ethyloxy)carbonyl,
j) ((1-methyl-1-(4-biphenylyl))ethyloxy)carbonyl,
k) (vinyloxy)carbonyl,
l) formly,
m) pivaloyloxymethyl and
n) diphenylphosphinyl;

5. The compound according to any one of the preceding claims wherein compound of Formula I contains exactly one **L².**

6. A compound of Formula II 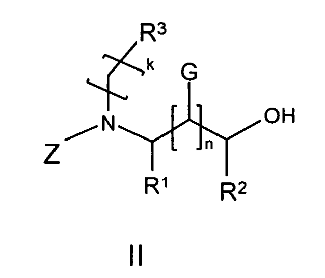 or pharmaceutically acceptable salts of an inorganic or organic acid thereof, hydrates, complexes, esters, amides, solvates or prodrugs thereof,
wherein
**R¹, R²** and **R³** are selected individually and independently from a group comprising:
a) hydrogen and
b) [F-18]-fluoro-(C₁-C₄)alkyl;
c) [F-18]-fluoro-(C₂-C₄)alkenyl and
d) [F-18]-fluoro-(C₂-C₄)alkynyl;
**G** is selected from the group comprising:
a) hydrogen,
b) [F-18]-fluoro,
c) [F-18]-fluoro-(C₁-C₄)alkyl,
d) [F-18]-fluoro-(C₂-C₄)alkenyl and
e) [F-18]-fluoro-(C₂-C₄)alkynyl;
**Z** is selected from the group comprising
a) hydrogen,
b) (C₁-C₄)alkyl
**k** is an integer of from 0 to 4;
**n** is an integer of from 0 to 3.

7. The compound according to claim 6 wherein compounds of Formula II contain exactly one [F-18]-fluoro atom.

8. The compound of formula II according to claims 6 or 7 for use as medicament or pharmaceutical.

9. The use of compound of Formula II for the manufacture of medicament or pharmaceutical for treatment.

10. The use according to claim 9 for treatment of cancer.

11. The compound of formula II according to claims 6 or 7 for use as diagnostic imaging agent.

12. The use of compound of Formula II for the manufacture of medicament or pharmaceutical for diagnostic imaging more preferably PET imaging.

13. The use according to claim 12 for imaging of cancer.

14. A pharmaceutical composition comprising compounds of Formula I or pharmaceutically acceptable salts of an inorganic or organic acid thereof, hydrates, complexes, esters, amides, solvates or prodrugs thereof.

15. A pharmaceutical composition comprising compounds of Formula II or pharmaceutically acceptable salts of an inorganic or organic acid thereof, hydrates, complexes, esters, amides, solvates or prodrugs thereof.

16. A compound of Formula III, 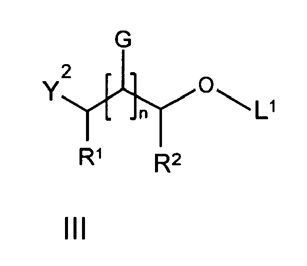 or pharmaceutically acceptable salts of an inorganic or organic acid thereof, hydrates, complexes, esters, amides, solvates or prodrugs thereof,
wherein
**Y²** is selected from the group comprising
a) -N(A²)-(CH₂₎ₖ-Q³ and
b) -N=C(U³)(U⁴);
**R¹, R²** and **R³** are selected individually and independently from a group comprising:
a) hydrogen,
b) [F-18]-fluoro-(C₁-C₄)alkyl,
c) [F-18]-fluoro-(C₂-C₄)alkenyl and
d) [F-18]-fluoro-(C₂-C₄)alkynyl;
**G** is selected from the group comprising:
a) hydrogen,
b) [F-18]-fluoro,
c) [F-18]-fluoro-(C₁-C₄)alkyl,
d) [F-18]-fluoro-(C₂-C₄)alkenyl and
e) [F-18]-fluoro-(C₂-C₄)alkynyl;
**A²** is selected from the group comprising
a) methyl,
b) ethyl and
c) L³;
**U³** and **U⁴** are selected individually and independently from the group comprising
a) aryl,
b) *tert*-butyl,
c) hydrogen,
d) methylsulfanyl,
e) halo-aryl,
f) hydroxyl-aryl,
g) nitro-aryl,
h) (C₁-C₄)alkyl-aryl and
i) ((C₁-C₄)alkyloxy)-aryl;
**L¹** is as defined above;
**L³** is as defined above;
**k** is an integer of from 0 to 4;
**n** is an integer of from 0 to 3.

17. A radiolabeling method for obtaining compound of formula III comprises the step of - Reacting a compound of formula I with a fluorinating agent for obtaining a compound of formula III.

18. The radiolabeling method of claim 17 wherein the fluorination agent is a fluorine radioactive isotope derivative, more preferably the fluorine radioactive isotope derivative is 4,7,13,16,21,24-Hexaoxa-1,10-diazabicyclo[8.8.8]-hexacosane K18F (crownether salt Kryptofix K18F), K¹⁸F, H¹⁸F, KH¹⁸F₂, Cs¹⁸F, Na¹⁸F or tetraalkylammonium salt of ¹⁸F.

19. A radiolabeling method for obtaining compound of formula III comprises the steps of
- Obtaining a compound of formula IV from a compound of formula V, and
- Reacting a compound of formula IV with a compound of formula VI,
wherein the compound of Formula IV is 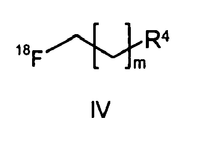 or pharmaceutically acceptable salts of an inorganic or organic acid thereof, hydrates, complexes, esters, amides, solvates or prodrugs thereof,
wherein
**m** is an integer from 0 to 4, and
**R⁴** is a leaving group,
the compound of Formula V is 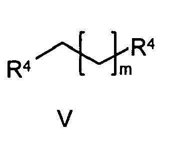 or pharmaceutically acceptable salts of an inorganic or organic acid thereof, hydrates, complexes, esters, amides, solvates or prodrugs thereof,
wherein
**R⁴** is defined as above, and
**m** is an integer from 0 to 4,
the compound of Formula VI is 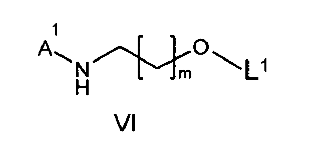 or pharmaceutically acceptable salts of an inorganic or organic acid thereof, hydrates, complexes, esters, amides, solvates or prodrugs thereof,
wherein
**A¹** is selected from the group comprising:
a) methyl,
b) ethyl and
c) L³ (protecting group);
**L³** is defined as above,
**L¹** is defined as above, and
**m** is an integer from 0 to 4.

20. The radiolabeling method according to claim 19 wherein R⁴ is selected from the group comprising:
a) iodo,
b) bromo,
c) chloro,
d) mesyloxy,
e) tosyloxy,
f) trifluormethylsulfonyloxy and
g) nonafluorobutylsulfonyloxy.

21. A radiolabeling method for obtaining compound of formula III comprises the steps of
- Reacting a compound of formula VII with fluorination agent.
wherein the compound of Formula VII is 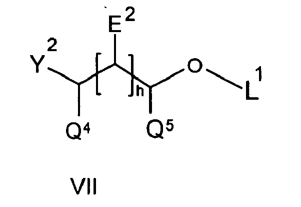 or pharmaceutically acceptable salts of an inorganic or organic acid thereof, hydrates, complexes, esters, amides, solvates or prodrugs thereof,
wherein
**Y²** is selected from the group comprising
a) -N(A³)-(CH₂)ᵤ-Q⁶ and
b) -N=C(U⁵)(U⁶)
**Q⁴, Q⁵** and **Q⁶** are selected individually and independently from the group comprising:
a) hydrogen and
b) L⁴-(C₁-C₄)alkyl;
c) L⁴-(C₂-C₄)alkenyl and
d) L⁴-(C₂-C₄)alkynyl;
**A³** is selected from the group comprising
a) methyl,
b) ethyl and
c) L³ (protecting group);
**U⁵** and **U⁶** are selected individually and independently from the group comprising
a) aryl,
b) *tert*-butyl,
c) hydrogen,
d) methylsulfanyl,
e) halo-aryl,
f) hydroxyl-aryl
g) nitro-aryl,
h) (C₁-C₄)alkyl-aryl and
i) ((C₁-C₄)alkyloxy)-aryl;
**E²** is selected from a group comprising
a) hydrogen,
b) L⁴,
c) L⁴-(C₁-C₄)alkyl,
d) L⁴-(C₂-C₄)alkenyl and
e) L⁴-(C₂-C₄)alkynyl;
**L¹** is defined as above;
**L⁴** is a leaving group and is selected from the group comprising
a) chloro,
b) bromo,
c) iodo,
d) mesyloxy and
e) tosyloxy;
**u** is an integer of from 0 to 4;
**h** is an integer of from 0 to 3.

22. The compounds of Formula IX 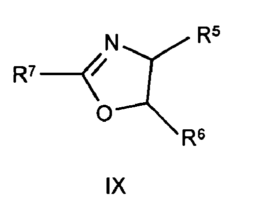 or pharmaceutically acceptable salts of an inorganic or organic acid thereof, hydrates, complexes, esters, amides, solvates or prodrugs thereof,
wherein
**R⁵** and **R⁶** are selected individually and independently from a group comprising:
a) hydrogen and
b) [F-18]-fluoro-(C₁-C₄)alkyl,
c) [F-18]-fluoro-(C₂-C₄)alkenyl and
d) [F-18]-fluoro-(C₂-C₄)alkynyl;
**R⁷** is selected from a group comprising:
a) (C₁-C₆)alkyl,
b) (C₁-C₆)alkenyl,
c) (C₁-C₆)alkynyl,
d) aryl and
e) ar-(C₁-C₆)alkyl.

23. The compound according to claim 21 wherein compound of Formula IX contains exactly one [F-18]-fluoro atom.

24. A compound of Formula X 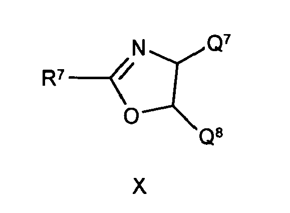 or pharmaceutically acceptable salts of an inorganic or organic acid thereof, hydrates, complexes, esters, amides, solvates or prodrugs thereof,
wherein
**Q⁷** and **Q⁸** are selected individually and independently from the group comprising:
a) hydrogen,
b) L²-(C₁-C₄)alkyl,
c) L²-(C₂-C₄)alkenyl and
d) L²-(C₂-C₄)alkynyl;
Wherein
**R⁷** and **L²** are defined as above.

25. The compound of claim 24 wherein the compound of Formula X contains exactly one L².

26. The radiolabeling method for obtaining compound of formula IX comprises the step of
- Reacting a compound of formula XI with fluorination agent
wherein
compounds of formula IX is as defined above,
the compound of Formula XI is 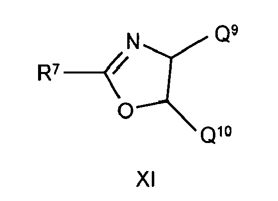 or pharmaceutically acceptable salts of an inorganic or organic acid thereof, hydrates, complexes, esters, amides, solvates or prodrugs thereof,
wherein
**Q⁹** and **Q¹⁰** are selected individually and independently from the group comprising
a) hydrogen;
b) L⁴-(C₁-C₄)alkyl;
c) L⁴-(C₂-C₄)alkenyl and
d) L⁴-(C₂-C₄)alkynyl;
**R⁷** and **L⁴** is defined as above.

27. The compound of claim 26 wherein compound of Formula XI contains exactly one L⁴_{.}

28. The radiolabeling method for obtaining compound of formula IX comprises the step of
- Reacting a compound of formula X with fluorination agent,
wherein compound of formula X is defined as above.

29. The radiolabeling method for obtaining compound of formula II comprises the steps of
- Radiolabelling of a compound of formula I or VII with a fluorination agent for obtaining a compound of formula III, and
- Deprotecting compound of formula III,
wherein compounds of formula I, II, III and VII are as defined above.

30. The radiolabeling method for obtaining compound of formula II comprises the steps of
- Radiolabeling of compound of formula V with a radioactive fluorination agent for obtaining a compound of formula IV, and
- Substituing compound of formula IV with compound of Formula VIII,
wherein compounds of formula II, IV, and V are as defined above.

31. A compound of Formula VIII 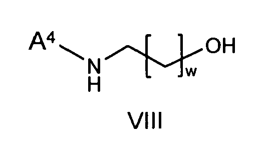 or pharmaceutically acceptable salts of an inorganic or organic acid thereof, hydrates, complexes, esters, amides, solvates or prodrugs thereof,
wherein **A⁴** is
a) hydrogen,
b) methyl or
c) ethyl,
**w** is an integer of from 0 to 3.

32. The radiolabeling method for obtaining compound of formula II comprises the steps of
- Radiolabelling of a compound of formula X or XI with a fluorination agent for obtaining a compound of formula IX, and
- Deprotecting compound of formula II,
wherein compounds of formula II, IX, X and XI are as defined above.

33. The compound of formula III or IX according to claims 16 and 22 respectively for use as medicament or pharmaceutical.

34. The use of compound of Formula III or IX according to claims 16 and 22 respectively for the manufacture of medicament or pharmaceutical for treatment.

35. The use according to claim 34 for treatment of cancer.

36. The compound of formula III or IX according to claims 16 and 22 respectively for use as diagnostic imaging agent.

37. The use of compound of Formula III or IX according to claims 16 and 22 respectively for the manufacture of pharmaceutical for diagnostic imaging more preferably PET imaging.

38. The use according to claim 37 for imaging of cancer.

39. A kit comprising a vial comprising a predetermined quantity of a compound having any one of the following general chemical Formulae or mixture thereof
a) compounds of Formula I;
b) compounds of Formula V and VI;
c) compounds of Formula V and VIII;
d) compounds of Formula VII;
e) compounds of Formula X or
f) compounds of Formula XI.
